# EUROPEAN PATENT SPECIFICATION

(11) **EP 3 523 655 B1**
(45) Date of publication and mention of the grant of the patent: **08.10.2025**
(21) Application number: 17855123.0
(22) Date of filing: 28.09.2017
(51) Int. Cl.: G01N 33/558, G01N 33/573, G01N 33/576

(54) **POINT OF CARE ASSAYS**
TESTS ZUR VERWENDUNG AM EINSATZORT
DOSAGES DE POINT D'INTERVENTION

(30) Priority: 30.09.2016 CN 201610878590
(43) Date of publication of application: 14.08.2019
(73) Proprietor: BioPoint Hong Kong Ltd, Wong Chuk Hang (HK)
(72) Inventor: ANDERSON, David Andrew, Melbourne, Victoria 3004 (AU); GARCIA, Mary Louise, Melbourne, Victoria 3004 (AU); VAN, Huy, Melbourne, Victoria 3004 (AU); ZHANG, Zhimei, Nanjing Jiangsu (CN)
(74) Representative: FRKelly
(86) International application number: PCT/IB2017/055943
(87) International publication number: WO 2018/060904

(56) References cited:
- WO-A2-2005/113761
- CN-A- 1 636 058
- DAVID ANDREW ANDERSON ET AL: "O-017: An immunochromatographic test for measurement of alanine aminotransferase 1 (ALT1) at point-of-care", JOURNAL OF VIRAL HEPATITIS., vol. 25, 12 June 2018 (2018-06-12), OXFORD, UK, pages 13 - 13, XP055691661, ISSN: 1352-0504, DOI: 10.1111/jvh.14_12922
- GARCIA MARY LUISE ET AL: "AN IMMUNOCHROMATOGRAPHIC TEST FOR MEASUREMENT OF ALANINE AMINOTRANSFERASE (ALT) AT POINT OF CARE", 16 May 2016 (2016-05-16), XP055691657, Retrieved from the Internet <URL:https://na.eventscloud.com/file_uploads/3c4f89406cb0ce889e4cdc0f7b4b20ff_075_DavidAnderson.pdf> [retrieved on 20200505]
- INGALILL RAFTER ET AL: "Isoform-specific alanine aminotransferase measurement can distinguish hepatic from extrahepatic injury in humans", INTERNATIONAL JOURNAL OF MOLECULAR MEDICINE, vol. 30, no. 5, 23 August 2012 (2012-08-23), GR, pages 1241 - 1249, XP055691652, ISSN: 1107-3756, DOI: 10.3892/ijmm.2012.1106
- LINDBLOM ET AL: "Isoforms of alanine aminotransferases in human tissues and serum-Differential tissue expression using novel antibodies", ARCHIVES OF BIOCHEMISTRY AND BIOPHYSICS, ACADEMIC PRESS, US, vol. 466, no. 1, 18 September 2007 (2007-09-18), pages 66 - 77, XP022258760, ISSN: 0003-9861, DOI: 10.1016/J.ABB.2007.07.023
- B. GLINGHAMMAR ET AL: "Detection of the mitochondrial and catalytically active alanine aminotransferase in human tissues and plasma", INTERNATIONAL JOURNAL OF MOLECULAR MEDICINE, vol. 23, no. 5, 1 May 2009 (2009-05-01), GR, pages 621 - 631, XP055500427, ISSN: 1107-3756, DOI: 10.3892/ijmm_00000173
- LI ET AL: "Preparation of Antibodies Against Human Alanine Transaminase I and Study Its Bionomics", 15 May 2010 (2010-05-15), XP009513947, Retrieved from the Internet <URL:1077952576> [retrieved on 20200505]
- HU , X.M. ET AL.: "Development of monoclonal antibodies and immunochromatographic lateral flow device for rapid test of alanine aminotransferase isoenzyme 1", PROTEIN EXPRESSION AND PURIFICATION, vol. 119, 22 November 2015 (2015-11-22), pages 94 - 101, XP029386711
- LI, Y.Q. ET AL.: "Prokaryotic expression of human I Alanine aminotransferase and Preparation of its antiserum", JOURNAL OF THE FOURTH MILITARY MEDICAL UNIVERSITY, vol. 30, no. 6, 31 December 2009 (2009-12-31), pages 530 - 533, XP009515838, ISSN: 1674-8913
- LINDBLOM, P. ET AL.: "Isoforms of alanine aminotransferases in human tissues and serum- Differential tissue expression using novel antibodies", A RCHIVES OF BIOCHEMISTRY AND BIOPHYSICS, vol. 466, 11 August 2007 (2007-08-11), pages 66 - 77, XP022258760
- GLINGHAMMAR, B. ET AL.: "Detection of the mitochondrial and catalytically active alanine aminotransferase in human tissues and plasma", INTERNATIONAL JOURNAL OF MOLECULAR MEDICINE, vol. 23, no. 5, 31 December 2009 (2009-12-31), pages 621 - 631, XP055500427
- SHEN, H.L. ET AL.: "Expression of truncated ALT1 protein and preparation of its polyclonal antibody", BASIC & CLINICAL MEDICINE, vol. 33, no. 3, 31 March 2013 (2013-03-31), pages 281 - 285, XP055595487
- LI, Y.Q.: "Preparation of Antibodies Against Human Alanine Transaminase I and Study Its Bionomics", CHINESE MASTER'S THESES FULL-TEXT DATABASE MEDICINE AND HEALTH SCIENCES, 15 May 2010 (2010-05-15), XP009513947
- YANG, R.Z. ET AL.: "Alanine Aminotransferase Isoenzymes: Molecular Cloning and Quantitative Analysis of Tissue Expression in Rats and Serum Elevation in Liver Toxicity", HEPATOLOGY, vol. 49, no. 2, 28 February 2009 (2009-02-28), pages 598 - 607, XP055500430
- LIAO, H.M. ET AL.: "Cloning, expression, purification and determination of enzymatic activity of human alanine aminotransferase(ALTl", JOURNAL OF SHENYANG PHARMACEUTICAL UNIVERSITY, vol. 27, no. 6, 30 June 2010 (2010-06-30), pages 489 - 494, XP009519722, DOI: 10.14066/j.cnki.cn21-1349/r.2010.06.002

## Description

### FIELD

The field of the specification is point of care testing and more specifically assays and kits for identifying abnormal levels of enzymes or abnormal liver function in subjects. Screening assays are also provided.

### BACKGROUND

Bibliographic details of references in the subject specification are also listed at the end of the specification.

Reference to any prior art in this specification is not, and should not be taken as, acknowledgement or any form of suggestion that this prior art forms part of the common general knowledge in any country.

Liver disease or hepatitis is a major healthcare burden worldwide. Liver disease can be caused by viruses (including hepatitis A, hepatitis B, hepatitis C, hepatitis D and hepatitis E viruses) or other infections, or by drug toxicity (including excessive alcohol use, and drugs used in treatment of conditions such as human immunodeficiency virus or tuberculosis infections), or through abnormal metabolic processes (including Non-Alcoholic Fatty Liver Disease [NAFLD] and Nonalcoholic Steatohepatitis [NASH]). For example, more than 360 million people have chronic Hepatitis B and more than 200 million have chronic hepatitis C, with high risks of persistent liver disease leading to liver fibrosis, cirrhosis and hepatocellular carcinoma, while increasing levels of obesity have led to large increases in the prevalence of NASH, now estimated to affect more than 25% of the population worldwide (Younossi *et al.,* 2016). Liver disease is also a major problem in management of pre-eclampsia, where monitoring of liver function in the mother is required in order to allow induction or surgical delivery of the neonate at the maximum gestational age possible but before liver disease becomes too severe, where it would threaten the health of both the mother and the baby.

Effective drugs are becoming more widely available for treatment of hepatitis B and hepatitis C, and the presence of infections such as HBV or HCV can be determined with serological tests for antibodies or antigens and/or molecular tests for viral DNA or RNA. Many therapies are also under development for conditions such as NAFLD and NASH, while prompt delivery of neonates can be effective in pre-eclampsia. However a major hurdle to effective use of these drugs, therapies and interventions is the identification of those patients who have the highest health risk and greatest need for therapy or intervention, because liver disease presents with non-specific signs and symptoms and cannot be diagnosed on clinical examination alone.

Liver disease is most conveniently detected and diagnosed by biochemical tests conducted on blood or plasma to detect the presence of abnormal amounts of liver-specific enzymes such as alanine aminotransferase (ALT), aspartate aminotransferase (AST) or gamma glutamyl transferase (GGT), or metabolites such as bilirubin. Using ALT as an example, the human ALT1 isoform is predominantly expressed in the cytosolic compartment of liver hepatocytes, and the destruction of hepatocytes during liver disease results in the release of soluble ALT into the plasma, where its enzymatic activity can be measured using a variety of biochemical test assay methods. In these methods, the enzymatic activity of the ALT converts a substrate into a product, and an instrument measures the change in color, absorbance or fluorescence of the substrate or product to calculate the concentration of ALT present in the sample. This concentration is then compared to the "normal", healthy levels expected under the test conditions and population being studied. These methods are well known in the art and are available in a wide range of automated and semi-automated analytical instruments that may use whole blood, plasma or serum as samples. An ALT test has also been described in which a visible color reaction of substrate can be detected in a POC device without the need for an instrument, but the visual dynamic range of this test limits its usefulness to identifying samples with highly elevated ALT levels (>3 times upper limit of normal, or 120 IU/L) (Pollock et al 2012). A more preferable test would be able to discriminate samples relative to the cutoffs recommended by the European Association for the Study of the Liver (EASL), which recommends an upper limit of 40 IU/L ALT for management of chronic hepatitis B and other conditions.

However a maj or limitation of these methods is the general need for instruments for two purposes; (a) to conduct the enzymatic reaction at a fixed temperature, since enzyme activity is highly temperature sensitive, and (b) to measure the enzymatic products at exact times or under kinetic conditions to provide precise measurements.

A further limitation in the case of ALT is the presence in humans of a second isoform, ALT2, which is more abundant in muscle tissues but is similarly released from damaged cells, for example following extreme exercise or heart failure. As the enzymatic assay does not distinguish between ALT1 and ALT2 elevated levels of ALT2 enzymatic activity in blood, plasma or serum may lead to a false diagnosis of liver disease. Even further, it is observed that ALT enzyme assays are not diagnostic in cases of severe disease where enzyme levels are diminished.

Hu *et al.* (2016) relates to the development of a pair of anti-ALT1 monoclonal antibodies (MAbs) of high specificity and affinity to prepare a Immunochromatographic lateral flow device (LFD) for rapid test of ALT1 in human serums. Lindbolm *et al.* (2007) relates to the development of isoenzyme specific ALT1 and ALT2 antibodies and the expression of the enzymes in human cells and organs.

Therefore there is an unmet need for liver function or disease tests that would be applicable to use at the point of care (POC) or near POC, to expand access to liver function testing for resource-poor settings worldwide, and for convenient use in clinics even in resourcerich settings. Here we describe, *inter alia* novel methods and approaches that have allowed the development of a POC test for ALT1 as a biomarker of liver disease, even severe liver disease, using the well-established technique of lateral flow immunochromatography, and with specificity for the ALT1 isoform rather than the ALT2 isoform. These methods will be generally applicable to diagnostic tests for other liver enzymes and other enzymes and metabolites thereof that are required for measurements at point of care where enzyme activities cannot be assessed, or more broadly as described herein. Although suitable for point of care, the subject methods can be used in any convenient immunoassay format.

### SUMMARY

Accordingly, in one aspect the specification (not according to the invention) provides an immunoassay suitable for but not limited to point of care to detect and quantify a liver enzyme in a blood sample from a subject. In one aspect, said assay comprises: (i) obtaining a blood sample from a subject, and (ii) detecting the presence and quantity of the liver enzyme in the sample. In particular, this is achieved by contacting the blood sample from a subject with a specific binding agent that specifically recognizes an epitope of the liver enzyme (antigen), that is not recognized by the majority of rodent antibodies when the rodent antibodies are in the presence of plasma. In one aspect, the binding agent is an antibody formed in a rabbit or other lagomorph or is an immunoglobulin domain thereof. The binding agent binds to the liver enzyme even in the presence of human plasma and forms an antigen-binding agent complex. Then the presence and amount of the complex is detected using a second binding agent linked to a detectable reporter. In some aspects, the specific binding agent and the second binding agent are the same. In the case of binding reagents that are polyclonal antibodies, the same preparation of polyclonal antibodies may be used for both antigen-binding and the detectable reporter.

In some aspects, the mass concentration of a liver enzyme, such as ALT1 or AST or GGT, is used to determine whether a subject can be safely treated by a general practitioner/in the community or whether the subject needs to be treated by a specialist such as a hepatologist or gastroenterologist. Thus, if ALT levels are supranormal (specifically, for example, over 40 IU/L or the equivalent mass concentration) the subject needs to be treated by a specialist. Whereas, if the liver enzyme levels are normal, the subject can be treated by a general practitioner/in the community. In some aspects, platelet levels are also assessed in the immune assay to provide a liver enzyme/platelet ratio, for example an APRI ratio (which is an AST: platelet ratio index, known in the art) as an indication of poor liver function/liver fibrosis.

As determined herein, rodent (murine) antibodies to human ALT1 are substantially blocked from binding human ALT1 by a component in human plasma. Accordingly, antibodies are employed in the present methods that that do not recognize immuno-dominant regions of the antigen or do not compete with rodent/murine antibodies against the antigen. Thus, antibodies are employed that recognize an epitope of a liver enzyme even in the presence of human plasma. In one aspect, this is achieved by developing antibodies in lagomorphs (rabbits). Suitable binding agents could also be developed by screening for binding in the presence of human plasma, as described herein. In another aspect, non-immunodominant parts of liver enzyme antigens are employed to generate antibodies that are not inhibited by human plasma. Suitable screening and development methods are known in the art.

In one aspect, the binding agent is an antibody or an antigen binding part thereof comprising an immunoglobulin domain known in the art, or another specific binding agent known in the art such as a ligand, receptor, chemical ligand, aptamer etc.

In one aspect, the specific antibody or binding agent recognizes human ALT1 and does not recognize human ALT2 or does not substantially recognize ALT2.

In one aspect, the blood sample can be whole blood, plasma or serum.

In one aspect, the liver enzyme is selected from alanine aminotransferase (ALT1), aspartate aminotransferase (AST) and gamma glutamyl transferase (GGT).

In one aspect, the mass concentration of liver enzyme is positively correlated with enzyme activity over a useful diagnostic range. This means that liver disease can be detected early when levels of liver enzymes are above the threshold for normal (a mass concentration equivalent to 40 IU/L in the case of ALT). Prior art assays are far less sensitive as ALT levels need to be 3-4 times higher than normal levels to be detectable by the assay.

The present invention provides an immunoassay suitable for point of care to assess liver disease or function in a blood sample from a subject, said assay comprising:
(i) i. contacting a blood sample from a human subject with a specific binding agent that recognizes an epitope of ALT1, that is not recognized by rodent antibodies raised against human ALT1 when the rodent antibodies are in the presence of human plasma, to form an antigen-binding agent complex and detecting the complex using a second or further binding agent linked to or comprising a detectable reporter; and
(ii) ii. detecting liver disease or liver function in the subject contingent upon the mass concentration of ALT1 in the sample determined from step (i);
wherein the specific binding agent is a lagomorph antibody or comprises an antigen binding part thereof, or wherein the specific binding agent is an antibody or antigen binding part thereof that recognizes the same epitope as the lagomorph antibody.

As determined herein, as the positive correlation between ALT enzyme activity levels and liver disease and recognized reference levels are established, although not for severe liver disease, now the finding that ALT1 mass concentration determined using the present methods is also positively correlated with ALT1 enzyme levels allows the user to determine liver disease in measurements of either mass concentration or equivalent enzyme levels. In subjects with severe liver disease or at risk of having or likely to have severe liver disease, the mass concentration provides a more accurate and quantitative assessment of liver disease, or the progress with treatments then enzyme activity levels. Accordingly, reference levels may be expressed as mass concentrations or enzyme activity unit, or rations as appropriate. The skilled person will appreciate that reference levels and suitable controls are determined using standard approaches as a routine development task. Thus for example a comparison of ALT1 mass concentration levels may be between subjects or groups of subjects to determine reference levels, a test result may be compared with reference levels for healthy coneols or with controls that have a liver disease and thus higher or lower comparative levels can be expected depending upon the reference level selected.

In one embodiment, the specific binding agent does not compete effectively with murine antibodies generated against the antigen/enzyme.

In one embodiment, the antibody is selected by screening for lack of inhibition of the reactivity against the liver enzyme by human plasma.

In one embodiment, the liver enzyme is selected from alanine aminotransferase (ALT1), but could also be aspartate aminotransferase (AST) and gamma glutamyl transferase (GGT). That is, the examples disclose the results of testing for ALT1 but AST or GGT could alternatively be tested.

In one embodiment, wherein the specific binding agent is a lagomorph antibody or comprises an antigen binding part thereof, or wherein the specific binding agent is a rabbit antibody or recognizes the same epitope as the rabbit antibody.

In one embodiment, the subject is human.

In one embodiment, the mass concentration of the liver enzyme or metabolite is positively correlated with the enzymatic activity for a subject without severe liver disease.

In one embodiment, and as determined herein using the subject method, the mass concentration of the liver enzyme or a metabolite thereof is positively correlated with increasing liver disease.

Generally a correlation of not less than about 70% will be effective. Levels of 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% are contemplated.

In one embodiment, very high mass concentrations of liver enzymes or a metabolite thereof is diagnostic of advanced liver disease or liver failure. In contrast, liver enzyme levels are often reduced in advanced liver disease and enzyme testing would lead to false negative tests for liver disease.

The present invention also provides an immunoassay suitable for point of care to assess liver disease or function, said assay comprising:
(i) contacting a blood sample from a human subject with a specific binding agent that specifically recognizes an epitope of ALT1, that is not efficiently recognized by rodent antibodies raised against human ALT1 when the rodent antibodies are in the presence of human plasma, to form an antigen-binding agent complex and detecting the complex using a second or further binding agent linked to a detectable reporter; and
(ii) detecting liver disease liver function in the subject contingent upon the mass concentration of ALT1 in the sample determined from step (i);
wherein the specific binding agent is a lagomorph antibody or comprises an antigen binding part thereof, or wherein the specific binding agent is an antibody or antigen binding part thereof that recognizes the same epitope as the lagomorph antibody.

In one embodiment, the specific binding agent is a rabbit antibody or comprises an antigen binding part thereof, or wherein the specific binding agent recognizes the same epitope as the rabbit antibody.

In one embodiment, the mass concentration of the ALT1 is positively correlated with ALT enzymatic activity for a subject without severe liver disease.

In one embodiment, the mass concentration of ALT1 is positively correlated with increasing liver disease.

In one embodiment, two or more liver enzymes or a metabolite thereof are assessed in the assay.

In one embodiment, reference levels equivalent to 40 IU/L ALT are contemplated. ALT activities of between 2 and 300 IU are contemplated including all integers in between.

In one embodiment, the binding agent is an antibody or an antigen-binding fragment thereof, an antigen-binding construct such as an affimer or aptamer, a ligand or binding part thereof.

In one embodiment, the assay is an enzyme-linked immunosorbent (ELISA)-type or immunochromatographic-type assay and the specific binding agent is immobilised on a support.

In one embodiment, the immunoassay is an ELISA- type or immunochromatographic-type, or microfluidic-type assay known in the art.

In one embodiment, the blood sample is contacted with the binding agents by applying the blood sample to a sample portion of an immunochromatographic or microfluidic device wherein the device sample portion is operably connected to spaced capture portions of the device and whereby the components of the sample flow from the device sample portion to and through the device capture portions, and wherein one capture portion comprises the binding agent which specifically binds to the antigen in the sample such that the antigen is captured by the binding agent to form a binding agent-antigen complex in the capture portion.

In one embodiment, the amount of antigen complex is detected using binding agents such as an antibody or antigen-binding fragment, ligand or affimer that specifically binds the antigen and directly or indirectly provide a detectable signal that can be quantified visually or photometrically including fluorometrically (by instrument reader).

In one embodiment visual quantitation is relative to an internal reference (a similar assay is disclosed in US Patent 8,409,818).

In one embodiment, the specific binding agents are conjugated to a detectable marker or microparticles comprising a detectable marker that provide a detectable signal.

In one embodiment, the capture portion is a test line.

In another aspect, platelet levels are assessed within the assay.

In one embodiment, the ratio of the visual or photometrically calculated signal from the antigen test line and the visual or photometrically calculated signal from the platelet marker test line provides a liver enzyme-platelet ratio index.

In one embodiment the liver enzyme-platelet ratio index provides a diagnosis of liver fibrosis. In one embodiment of this assay, the liver enzyme is ALT1 or AST.

In one embodiment the present invention provides a kit for measuring the mass concentration of ALT1 or for detecting liver disease comprising (i) a chromatographic device comprising a porous membrane operably connected to a sample portion, one or more capture (test) portions, and optionally one or more of the following; a conjugate (detection marker) portion, a sucker portion, a suitable control portion and optionally a cell lysis or solubilisation portion, and (ii) a lagomorph antibody that recognizes an epitope of ALT1 and forms an ALT1-rabbit antibody complex or a binding agent comprising the antigen binding component thereof, and (iii) optionally instructions for using the device to determine liver function.

In one embodiment, the device is suitable for reverse or lateral flow immunochromatographic formats.

In another embodiment, the present invention provides a kit comprising (i) a chromatographic device comprising a porous membrane or a microfluidic device operably connected to a sample portion, two or more capture (test) portions, and optionally one or more of the following; a conjugate (detection marker) portion, a sucker portion, a suitable control portion and optionally a cell lysis or solubilisation portion, and (ii) a specific binding agent that recognizes an epitope of ALT1 or other liver enzyme or metabolite thereof, that is not recognized by or in competition with rodent antibodies when the liver enzyme or metabolite are in the presence of human plasma, and forms a liver enzyme-antibody/binding agent complex, and a second binding agent that binds specifically to platelets in the sample and forms a platelet marker-binding agent complex wherein the binding agents are either immobilised to separate capture portions and/or contained within conjugate portions and (iii) optionally instructions for using the device to determine the liver enzyme-platelet ratio index as a measure of liver function/fibrosis and wherein the specific binding agent is a lagomorph antibody or comprises an antigen binding part thereof, or wherein the specific binding agent recognizes the same epitope as the lagomorph antibody.

In one embodiment the ratio developed is the AST/platelet ratio or the ALT1/platelet ratio, or combinations thereof.

The kit may be a reverse or lateral flow immunochromatographic format.

The binding agent may comprise an immunoglobulin domain.

The kit may employ any one of the assays described herein or known variants thereof.

In one aspect (not according to the invention), the specification provides a method of treating liver disease, the method comprising (i) assessing the mass concentration of ALT1 in a blood sample from a subject comprising contacting a blood sample from a human subject with a lagomorph antibody that specifically recognizes an epitope of ALT1 in the presence of plasma to form an antigen-antibody complex and detecting the complex using a second binding agent linked to a detectable reporter, and (ii) recommending a treatment program or treating the subject if the level of ALT1 exceeds a normal/control ALT1 level.

In one aspect (not according to the invention), the specification provides a method of treating liver disease, the method comprising (i) assessing the mass concentration of ALT1 in a blood sample from a subject comprising contacting a blood sample from a human subject with a lagomorph antibody that specifically recognizes an epitope of ALT1 in the presence of plasma, or a binding agent comprising the antigen binding component thereof, to form an antigen-antibody complex and detecting the complex using a second or further (eg, third) binding agent linked to or comprising a detectable reporter, and (ii) recommending a treatment program or treating the subject contingent upon the observed level or mass concentration of ALT1 in the subject.

In one aspect (not according to the invention), the method is sensitive and able to detect a mass concentration of human ALT1 equivalent to about 40 IU/L ALT or between 20 and 200 IU/L ALT.

Suitable treatments are known in the art and include anti-viral agents, dietary changes, physical exercise, yoga, anti-obesity drugs, glucose control agents, lipid lowering drugs, cytoprotective agents, anti-diabetes medication such as anti-TNF monoclonal antibodies, adenosine system drugs, renal transport glucose blockers such as PPAR agonists, herbal products, antihypertensive agents, peripheral cannabinoid agonists, thyroid hormone analogues, farnesoid X receptor agonists, insulin sensitizing agents, iron depletion drugs, among others.

Conditions affecting the liver include, without limitation hepatitis, pre-eclampsia, drug toxicity, metabolic disease, non-alcoholic fatty liver disease, non-alcoholic steatohepatitis, liver fibrosis, cirrhosis, hepatocellular carcinoma etc.

In another aspect(not according to the invention), the present specification provides a method of screening for binding agents that are substantially not inhibited from antigen binding by plasma: said method comprising contacting antigen and a potential antigen binding agent in the presence of different concentrations of plasma to determine whether the binding agent can bind the antigen in the presence of plasma.

In one aspect the binding agent is an antibody, monoclonal antibody or antigen binding part thereof, peptide ligand, nucleic acid binding ligands.

In one aspect, methods of selecting antibodies suitable for the subject assays are determined by screening for lack of substantial inhibition of their reactivity against ALT by human plasma. In this way antibody reagents are developed from any species including mouse, rat, rabbit that are not affected by plasma (i.e., they recognise non-immunodominant sites that are not blocked by plasma).

In one aspect (not according to the invention), the present specification provides a method of selecting binding reagents other than antibodies suitable for use in the assays as described herein, by screening for lack of substantial inhibition of their reactivity against ALT1 by human plasma. In one aspect convenient binding agents include developing protein ligands by phage display or related methods, nucleic acid aptamer ligands, chemical ligands etc.

In one aspect (not according to the invention), the specification provides a method of screening for antibody or other specific binding agents that are substantially not inhibited from antigen binding by plasma, the method comprising contacting antigen and a potential antigen binding agent in the presence of different concentrations of plasma to determine whether the binding agent can bind the antigen in the presence of plasma.

In one aspect, the antigen is a liver enzyme such as ALT1.

### BRIEF DESCRIPTION OF THE FIGURES

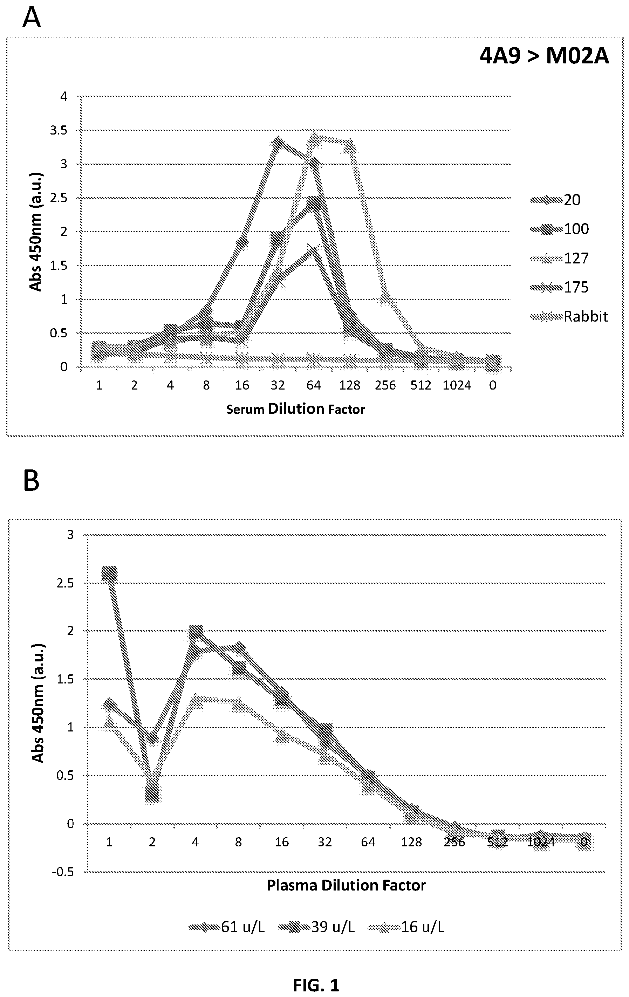

**Figure 1** illustrates ELISAs showing poor correlation of apparent ALT mass versus ALT enzymatic activity and the effect of plasma dilution. Upper panel: ELISA using monoclonal antibody 4A9 capture, monoclonal antibody M02A detection, for four human plasma samples with different enzymatic ALT levels, or rabbit serum as negative control. Note strong inhibition of antigenic reactivity in less diluted samples (bell-shaped curve or prozone) and lack of correlation between enzymatic ALT levels and antigenic reactivity observed. Lower panel: Commercial Elabscience ELISA kit for ALT, for three human plasma samples with different enzymatic ALT levels. Note variable inhibition of antigenic reactivity in less diluted samples (bell-shaped curve or prozone) and lack of correlation between enzymatic ALT levels and antigenic reactivity observed.

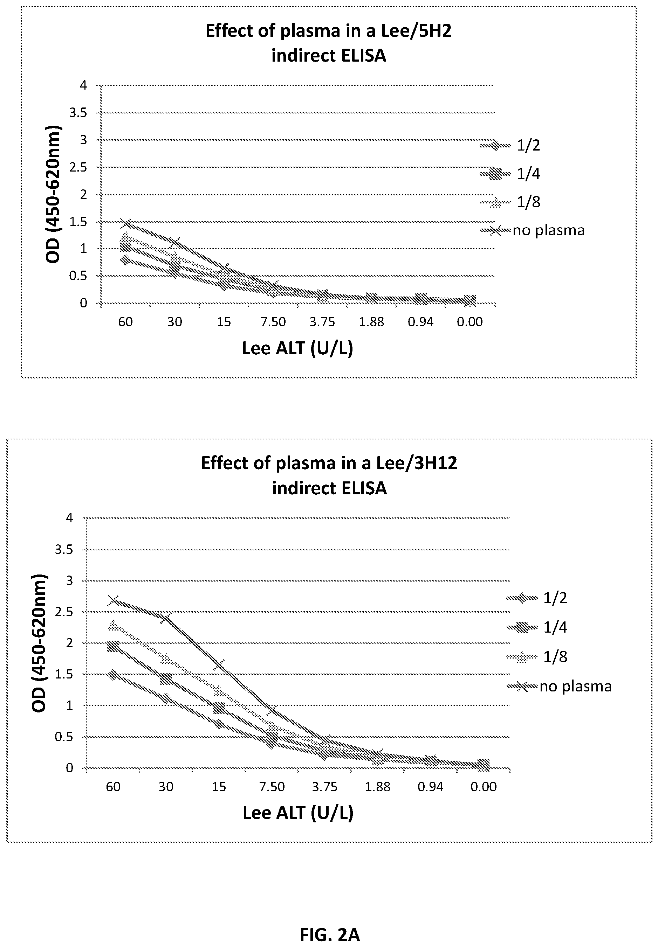

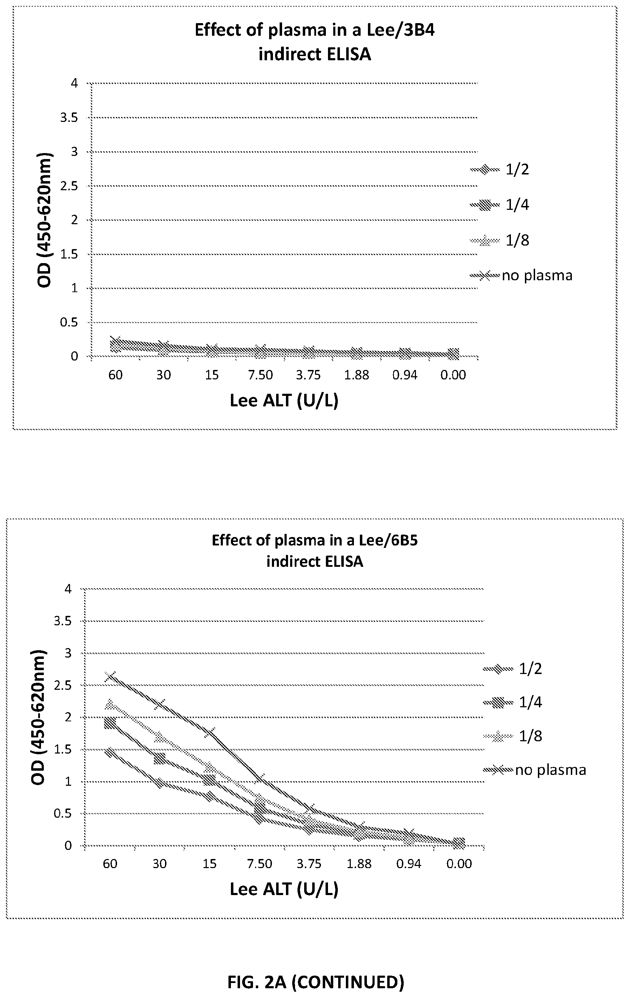

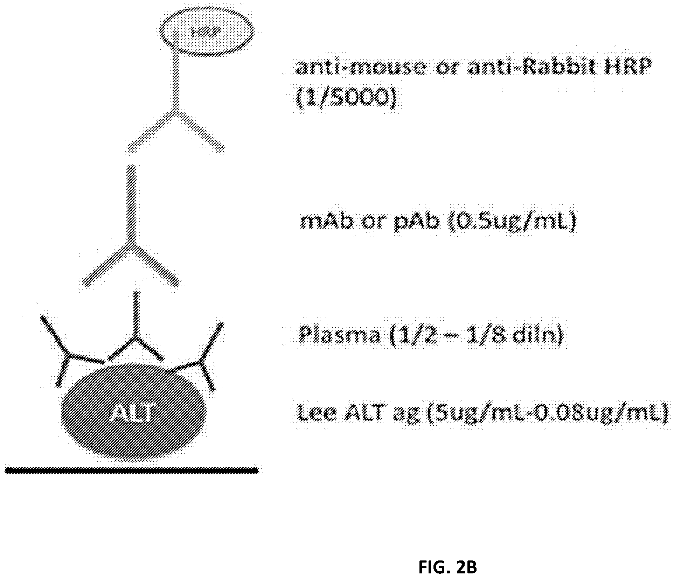

**Figure 2** illustrates ELISAs showing inhibition of mouse monoclonal antibody reactivity against ALT (Lee Bioscience) that was coated directly on ELISA plates, depending on the concentration of human plasma also added. Each of the antibodies shown demonstrated sensitivity to the presence of patient sera even at 1:8 dilution of sample. Similar results were observed for 12 other mouse monoclonal antibodies against ALT.

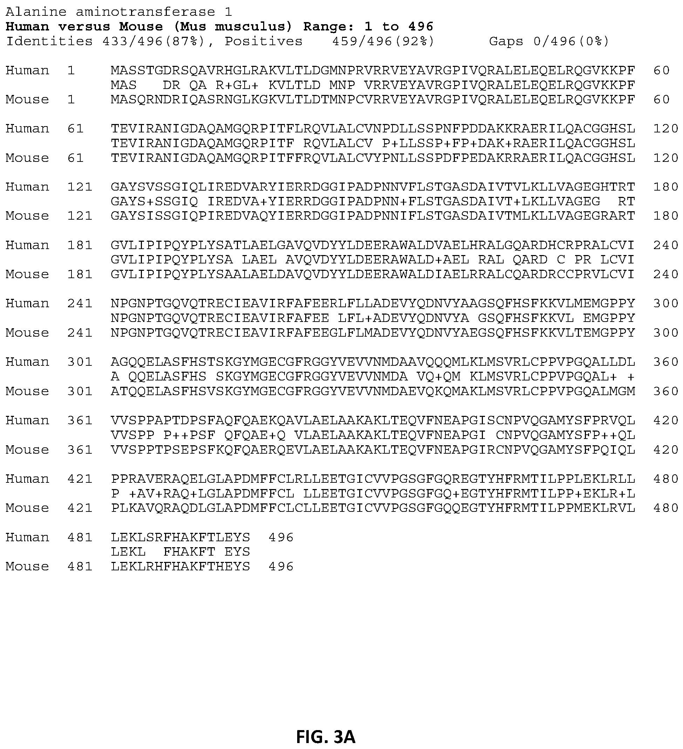

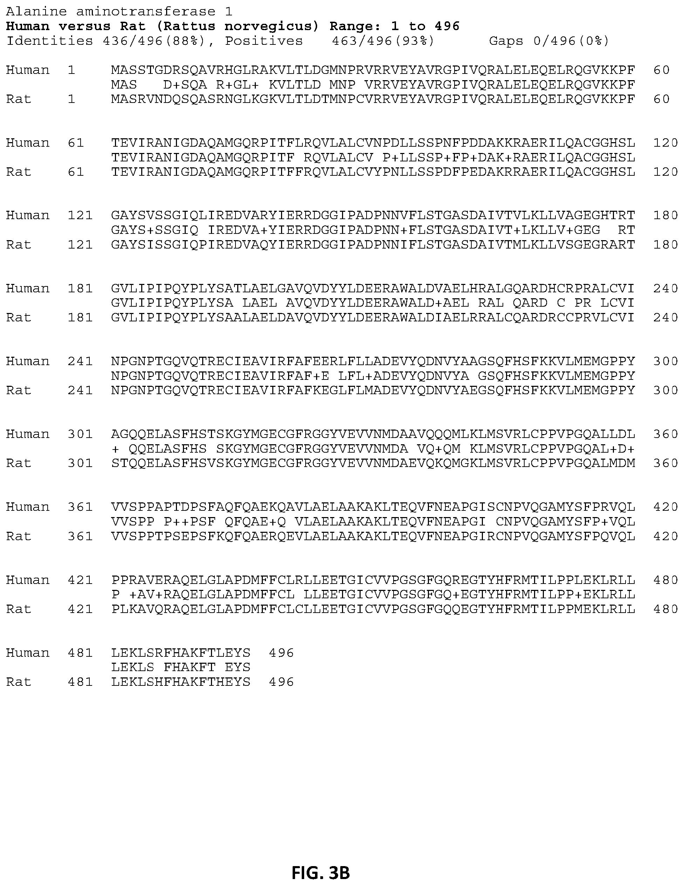

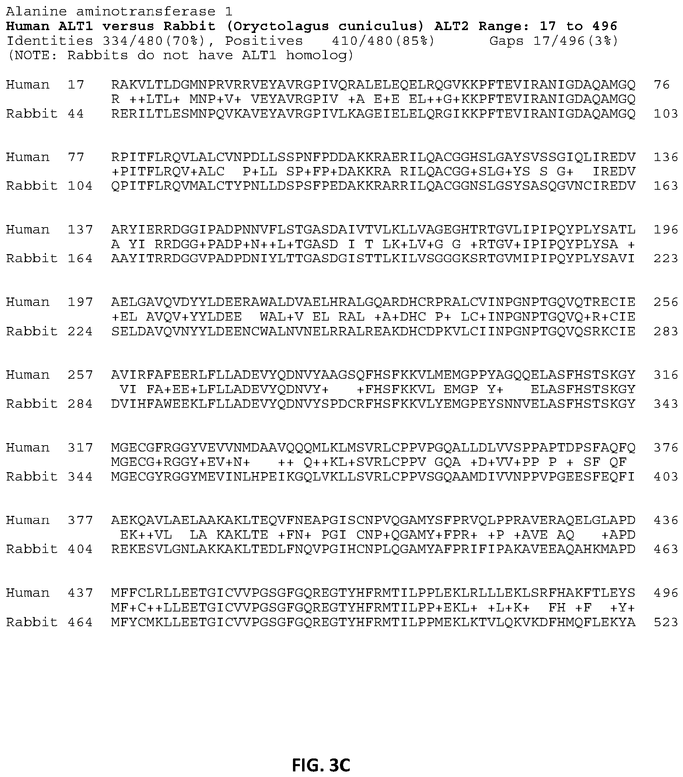

**Figure 3** **(A-C)** provides a comparison of the amino acid sequence of ALT1 in humans and in mice. Alignment of the sequences for human ALT1 and ALT1 from mouse (Figure 3A), rat (Figure 3B) or ALT2 from Rabbit (Figure 3C), noting that rabbits do not have a close homolog of the ALT1 gene and express only ALT2. Note the close identity and similarity of ALT1 from humans, mice and rats, whereas rabbit ALT2 is only 70% identical leading to improved immunogenicity of the many regions of difference compared to human ALT1.

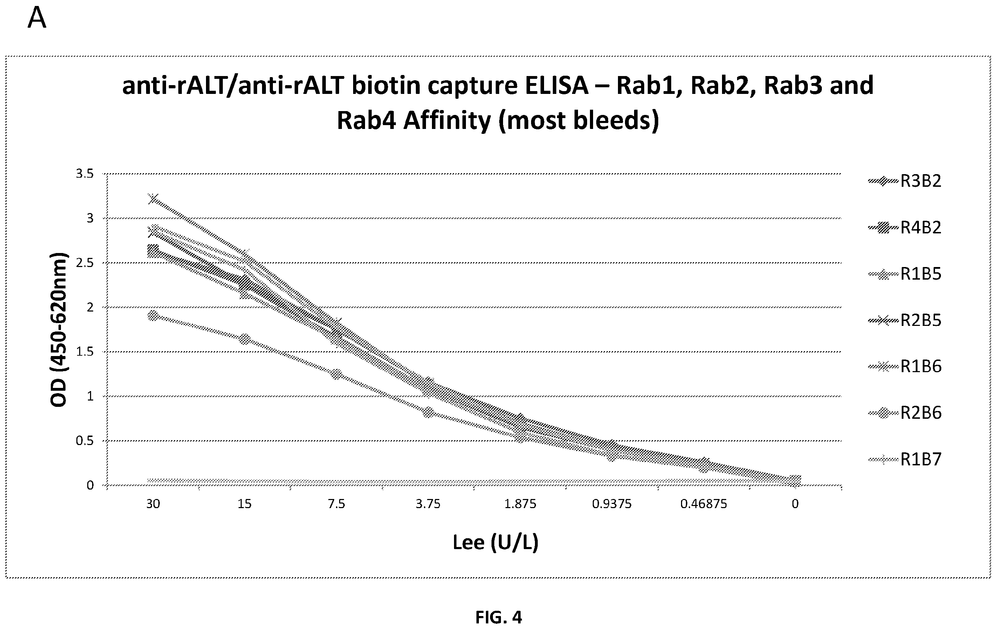

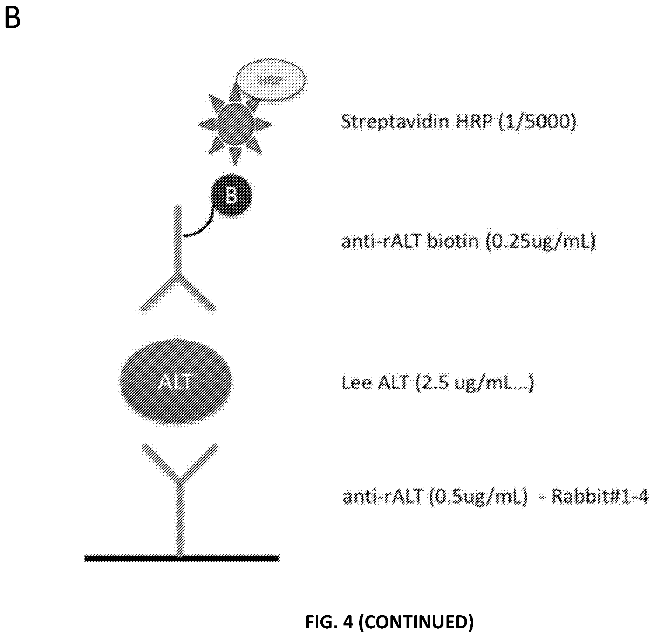

**Figure 4** illustrates that rabbits produced robust antibody responses after immunization with purified human ALT1. Rabbits immunised with recombinant ALT1 demonstrate high levels of antibody in their serum (not shown) or purified IgG. After labelling of the purified IgG with biotin, an ELISA using the format shown was used to compare the ability of each of the rabbit test bleeds to capture and detect ALT (Lee Bioscience). Note that all rabbit samples were able to capture and detect the Lee ALT when diluted in laboratory buffers without plasma.

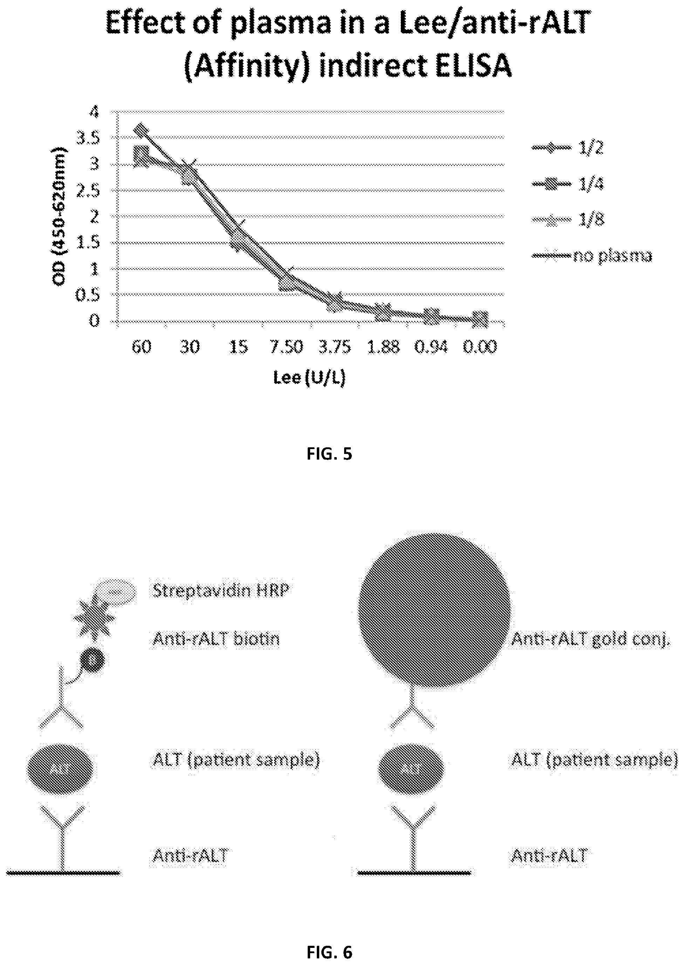

**Figure 5** illustrates using the same ELISA format as Figure 2, we demonstrate that rabbit polyclonal antibodies reactivity against ALT are not affected by the presence of human plasma at high concentrations (1:2, 1:4 or 1:8 dilutions of plasma give the same reactivity as no added plasma).
**Figure 6** illustrates a schematic of the detection system utilized in a lateral-flow, immunochromatographic test strip.

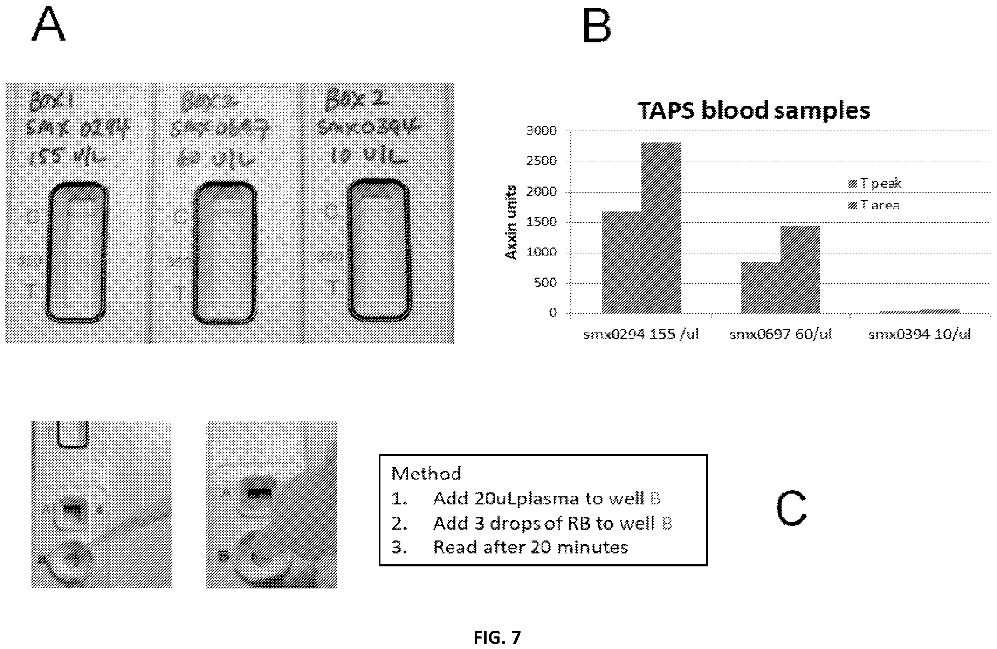

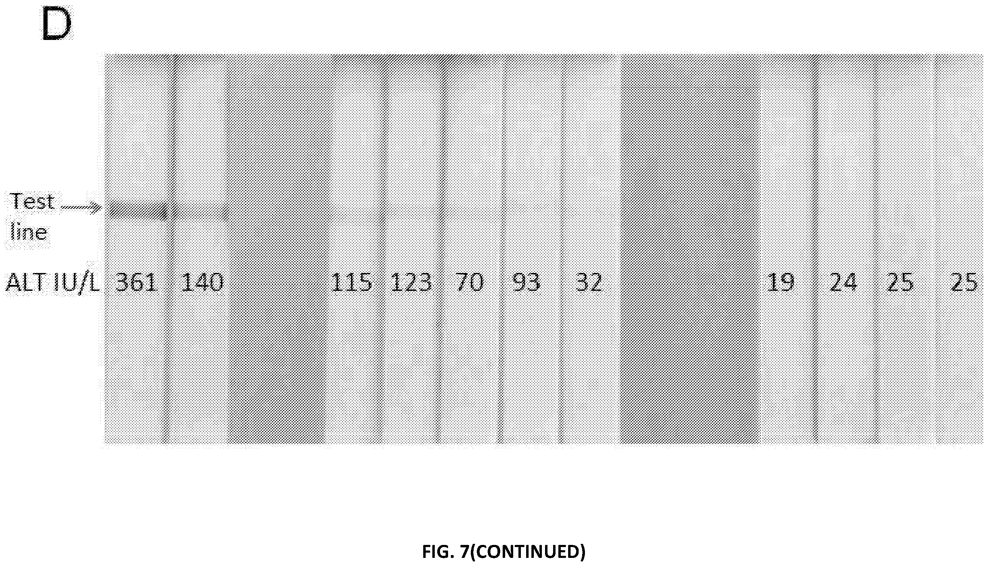

**Figure 7** illustrates examples of lateral flow immunochromatographic test for detection of ALT in plasma or whole blood. Upper panel; photos of test strips run in cassettes and showing different levels of test activity (line adjacent to "350" label) for three samples with 155 U/L, 60 U/L or 10 U/L ALT. Middle panel: assay method. Lower panel: Visual correlation of ALT enzymatic activity (U/L) versus intensity of test line reactivity for patient samples with >30 U/L (n=7) and only faint reactivity for lower ALT levels (n=4).

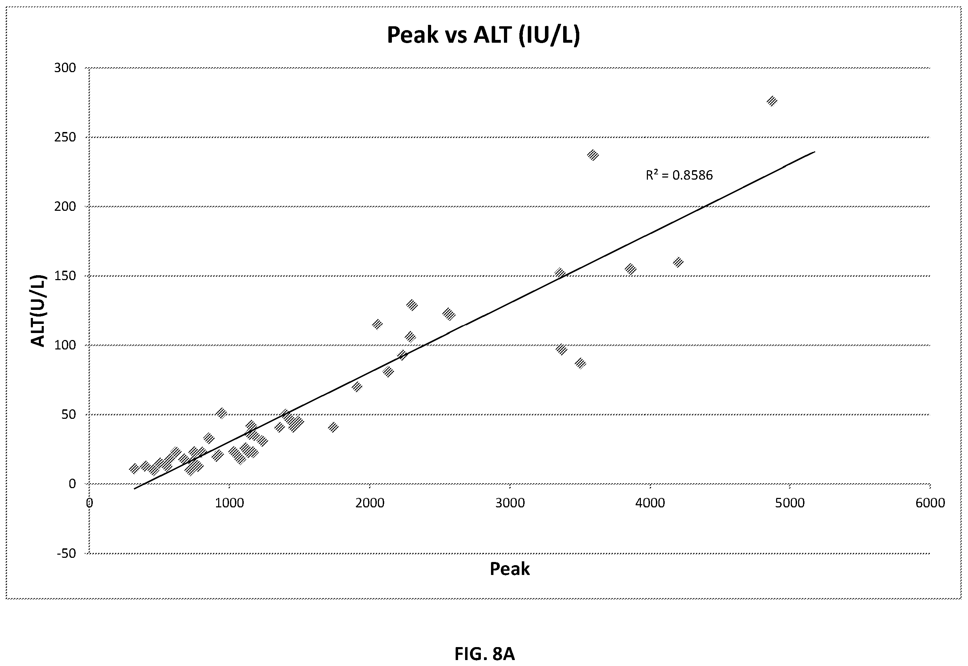

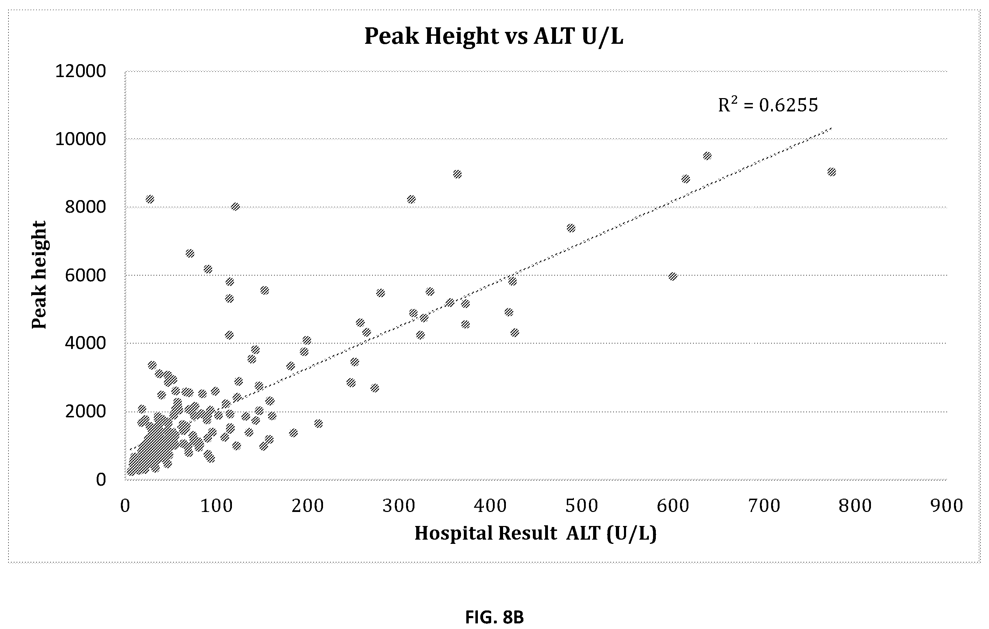

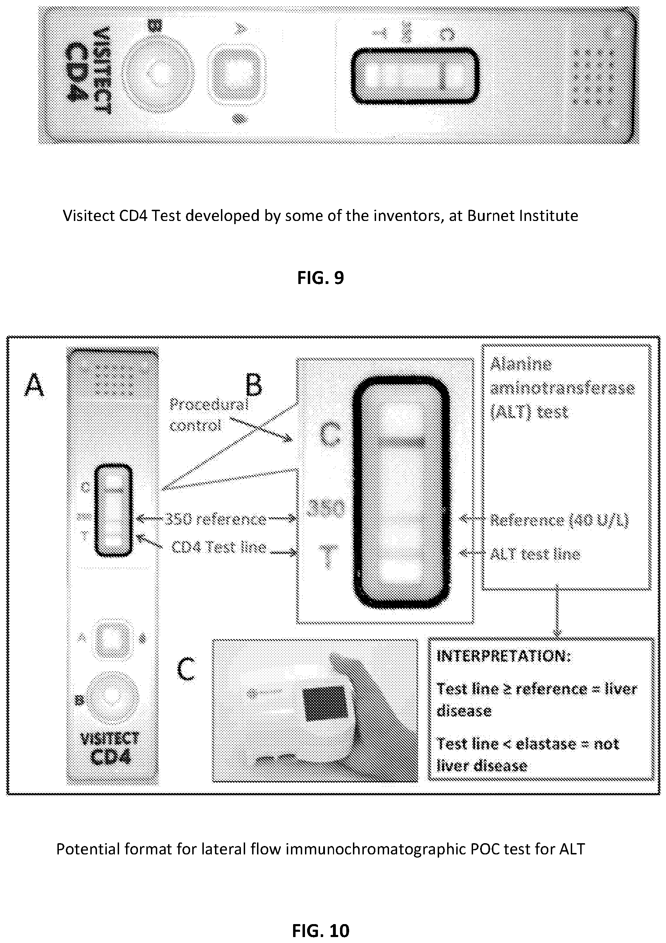

**Figure 8A-B** illustrate measurement of test line activity using the AX-2 instrument (shown in Figure 10C) demonstrates significant correlation between ALT enzymatic activity (U/L) and the test line peak height (Peak). Samples from hepatitis patients in Melbourne (n=48, Figure 8A) or Nanjing (n=174, Figure 8B) both demonstrate significant levels of correlation, with some samples showing higher peak heights than expected from the overall correlation, consistent with elevated levels of total ALT versus enzymatic ALT in more progressive liver disease.
**Figure 9** illustrates the CD4 T-cell test, the amount of CD4 antigen represented at the test (T) line is directly proportional to the number of CD4 T-cells. The test signal is then compared visually or by instrument reader versus one or more reference lines representing clinically relevant levels of CD4 T-cells, in the figure the example of 350 T-cells per µl which is recommended by WHO for prioritisation of antiretroviral therapy in HIV infection. Similarly, for ALT reference line is employed corresponding to one or more clinically relevant amounts of ALT, including the 40 IU/L level recommended by EASL. A schematic of the proposed semi-quantitative ALT test is shown in Figure 10.
**Figure 10** illustrates the potential format for lateral flow immunochromatographic POC test for ALT. Visitect CD4 test (A), expanded view and schematic of proposed ALT test (B, red boxes), and Axxin AX-2 reader (C). For visual interpretation an intensity for the ALT line greater than the Reference line might be considered to be diagnostic of ALT levels above a cutoff such as 40 IU/L, however with the use of a reader such as the Axxin AX-2 reader (Figure 10C) a fully quantitative result can be determined.

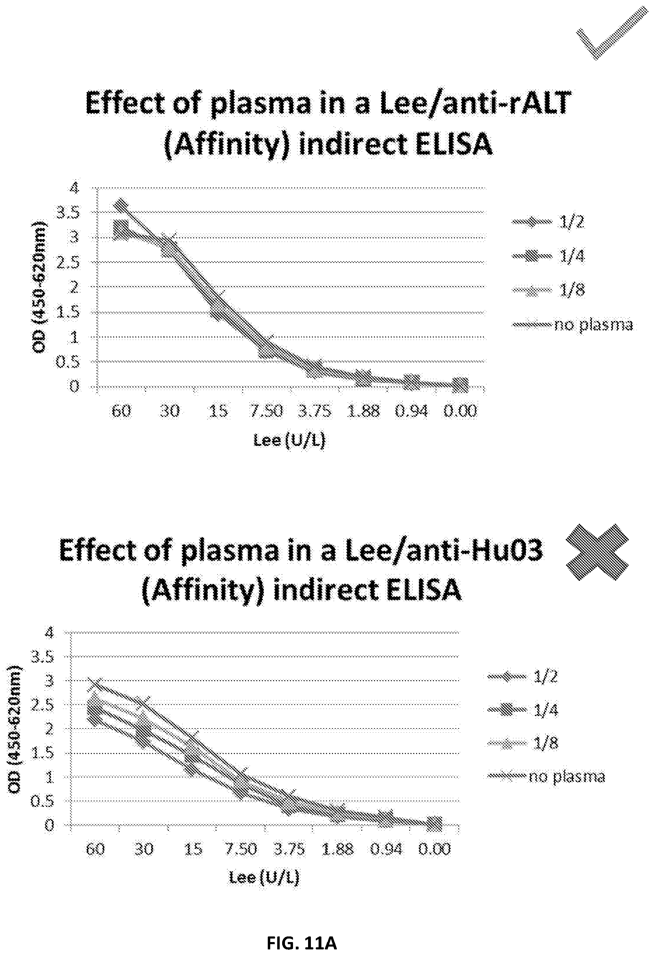

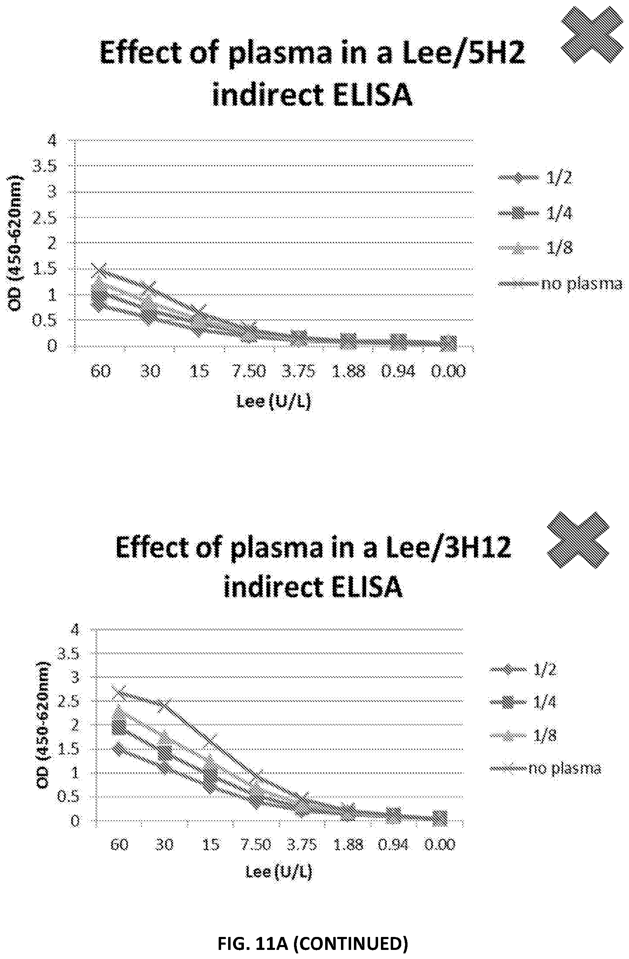

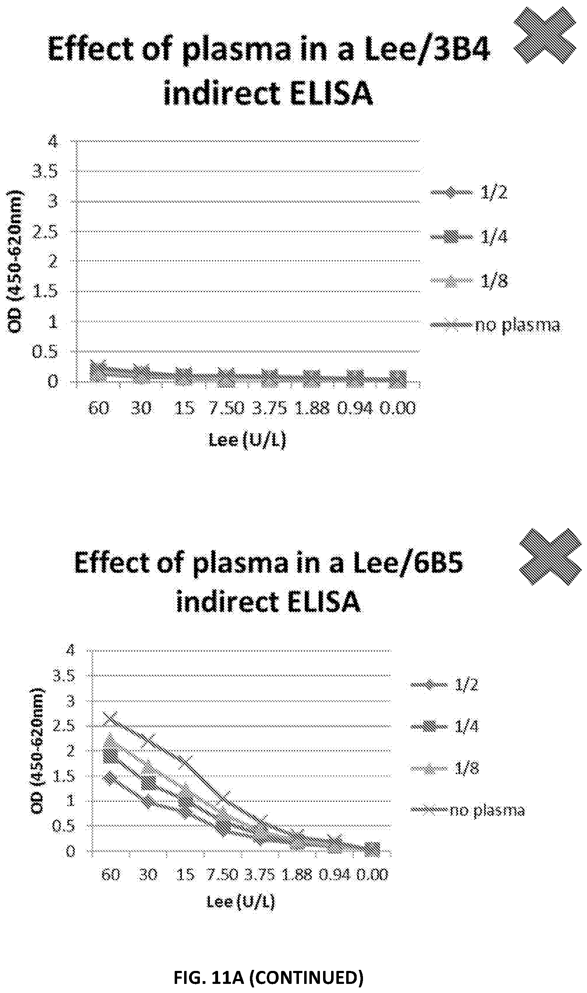

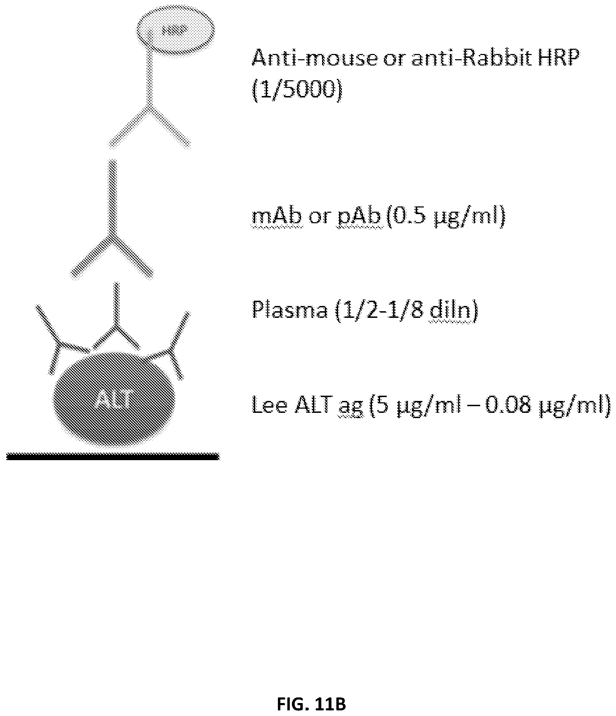

**Figure 11A-B** illustrate the design of ELISAs that can be used to identify polyclonal antibodies, monoclonal antibodies or other binding agents that are suitable for detection of ALT1 in human plasma samples. Using the format shown schematically in Figure 11B, ALT is coated on an assay surface followed by various dilutions of human plasma, before addition of the binding agent (e.g. antibody) and then detection reagents. Comparison of the amount of binding in the presence of plasma versus no plasma can demonstrate that binding reagents are suitable for the purpose, as shown in the upper left panel of Figure 11A for rabbit anti-rALT (affinity purified) with no effect by plasma, versus reagents that are unsuitable for the purpose as shown for a range of monoclonal antibodies in the remaining panels, where there was variable levels of inhibition in the presence of plasma (Hu03, 5H2, 3H12, 6B5 monoclonal antibodies) or poor reactivity even in the absence of plasma (3B4 monoclonal antibody).

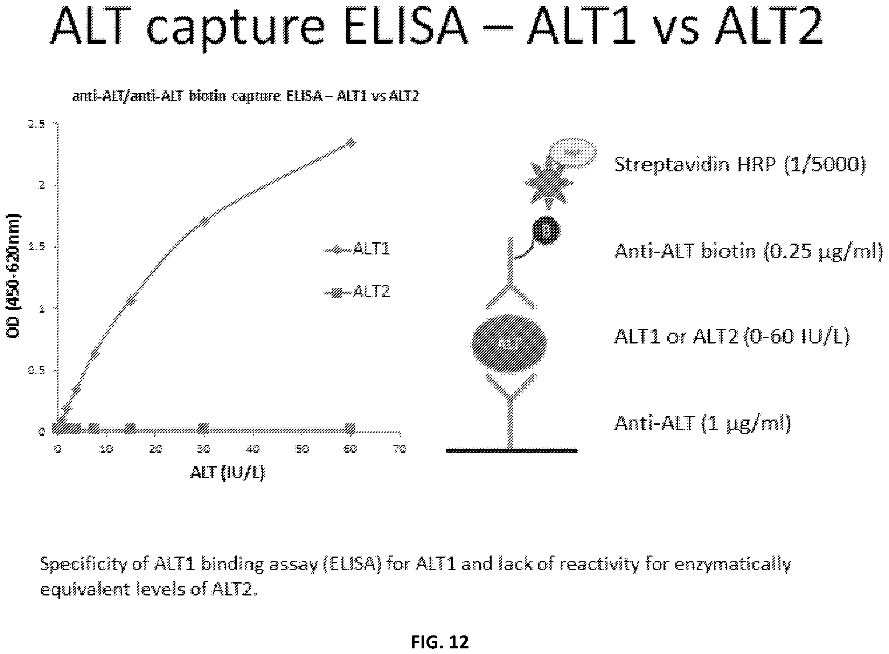

**Figure 12** illustrates the specificity of the ALT1 binding assay for ALT1 and the lack of reactivity for enzymatically equivalent levels of ALT2 in an ELISA assay. Unpurified human ALT1 or human ALT2 (SEQ ID: 6) representing equivalent levels of enzymatic ALT activity are coated on ELISA plates followed by rabbit antibodies against ALT1, ELISA reactivity is only seen for ALT1 and not ALT2 for all concentrations of enzymatic ALT, demonstrating specificity of the reagent and the assay for ALT1 and not ALT2.

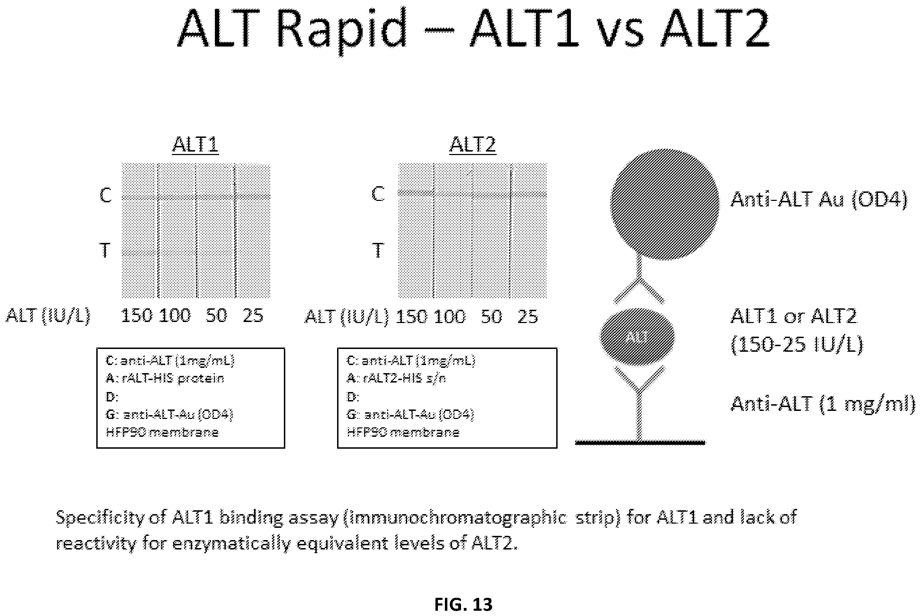

**Figure 13** illustrates the specificity of the ALT1 binding assay for ALT1 and the lack of reactivity for enzymatically equivalent levels of ALT2 in an immunographic strip assay. Unpurified human ALT1 or human ALT2 (SEQ ID: 6) representing equivalent levels of enzymatic ALT activity are subjected to the immunochromatographic assay as in Figure 7, only ALT1 is detected in the immunochromatographic test.

### KEY TO SEQUENCE LISTING

SEQ ID NO: 1 amino acid sequence of human alanine aminotransferase 1 (residues 1 - 496)
SEQ ID NO: 2 amino acid sequence of mouse alanine aminotransferase 1
SEQ ID NO: 3 amino acid sequence of rat alanine aminotransferase 1
SEQ ID NO: 4 amino acid sequence of human alanine aminotransferase 1 (residues 17 - 496)
SEQ ID NO: 5 amino acid sequence of rabbit alanine aminotransferase 2
SEQ ID NO: 6 amino acid sequence of human alanine aminotransferase 2

### DISCUSSION OF EMBODIMENTS

The subject disclosure is not limited to particular screening procedures for agents, specific formulations of agents and various medical methodologies, as such may vary.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements.

Reference herein to an "antibody" includes a whole antibody or an antigen binding part of an antibody, an immunoglobulin domain of an antibody as known in the art.

As used herein the singular forms "a", "an" and "the" include plural aspects unless the context clearly dictates otherwise. Thus, for example, reference to "a composition" includes a single composition, as well as two or more compositions; reference to "an agent" includes one agent, as well as two or more agents; reference to "the disclosure" includes single and multiple aspects of the disclosure and so forth.

Unless defined otherwise, all technical and scientific terms used herein have the same meanings as commonly understood by one of ordinary skill in the art to which this disclosure belongs. Any materials and methods similar or equivalent to those described herein can be used to practice or test the present disclosure. Practitioners are particularly directed to and Ausubel et al., Current Protocols in Molecular Biology, Supplement 47, John Wiley & Sons, New York, 1999; Colowick and Kaplan, eds., Methods In Enzymology, Academic Press, Inc.; Weir and Blackwell, eds., Handbook of Experimental Immunology, Vols. I-IV, Blackwell Scientific Publications, 1986; for definitions and terms of the art and other methods known to the person skilled in the art.

The clinical utility of tests for ALT and other liver disease markers is well demonstrated by the large number of instruments and tests available and in widespread use, but all these tests are designed for use in laboratory or clinical facilities with reliable power supplies, trained technicians and other infrastructure that are commonly lacking in resource-poor settings. Furthermore, transportation of blood or plasma to centralized facilities for testing is reliant on fast, refrigerated transport to maintain the enzymatic activity of the samples.

Kim *et al.,* (2009) reported tests in the format of enzyme-linked immunosorbent assays (ELISAs) for ALT mass concentration using combinations of monoclonal antibodies (MAbs) produced in mice. However a striking feature of the work by Kim *et al.* is the observation that under the conditions of their test, there was very low correlation between ALT enzymatic activity and mass concentration. Similarly, Hu *et al* (2016) reported a lateral-flow, immunochromatographic test for ALT1 using combinations of monoclonal antibodies (MAbs) produced in mice where there was also very low correlation with enzymatic ALT levels.

The biochemical tests are not accurate in subjects with severe or progressed liver disease and enzyme function is reduced. The authors of Kim *et al.* observed that despite low correlation with enzymatic ALT, there was good utility of the ALT ELISA for detecting patients with more severe forms of viral hepatitis (cirrhosis and hepatocellular carcinoma), where the ALT mass concentration was increased but the amount of enzymatic ALT was somewhat reduced. The major need for liver disease testing is to identify patients before they reach such advanced stages of liver disease, for example EASL recommends an upper limit of 40 IU/L ALT for management of chronic hepatitis B, and similar needs exist for liver enzyme tests in drug toxicity, pre-eclampsia and other acute or chronic conditions.

In addition, healthcare workers will have an expectation that a new ALT test should show a reasonable level of correlation with the established technology of enzymatic testing. Furthermore it will be appreciated that in accordance with the present disclosure, detection of excessive amounts of ALT mass concentration provides additional clinical utility in more advanced forms of liver disease, such as chronic viral hepatitis. The immunochromatographic test for ALT1 as described by Hu *et al* (2016) would not meet this expectation because the authors describe an assay that exhibits a very low correlation with enzymatic ALT levels.

Commercially-available ELISAs for measurement of ALT in human plasma also exhibit very poor correlation with enzymatic ALT levels, and surprisingly yield results that are highly dependent on the sample dilution used for serum or plasma (see Figure 1).

Immunoassays are a useful form of assay that exploit the specificity, strength and diversity of antibody-antigen reactions to analyze samples and detect specific components therein. A wide range of immunoassay techniques are available, such as those classically described in Wild D. "The Immunoassay Handbook" Nature Publishing Group, 2001 and subsequent innovations. More recent innovations are in the area of antigen binding fragments comprising immunoglobulin domains and derivatives thereof. Peptide aptamers, affimers, and chemical ligands are also contemplated and provide advantages in the area of production and attributes such as specificity, longevity/stability and sensitivity.

Lateral flow assays and more recently non-lateral flow and microfluidics provide a useful set up for biological assays. Such assays can be qualitative, quantitative or semi quantitative. In microfluidic devices, small volumes of liquid are moved through microchannels generated in, for example, a chip or cartridge. A wide range of detection reagents are available including metal nanoparticles, coloured or luminescent materials. Resonance enhanced adsorption (REA) of bioconjugated metal nanoparticles offers rapid processing times and other advantages. Devices may be combined with barcode technologies to identify the patient and the analyte being tested. Computer software and hardware for assessing input data are encompassed by the present disclosure.

A wide range of methods for the detection of antibody to specific antigens/enzymes are also known. For example, the enzyme-linked immunosorbent assay (ELISA), Western and dot blot assays, and radio-immunoassay (RIA) are routinely used in laboratories. Arrays and high throughput screening methods are also employed.

Qualitative assays providing an intermediate or definitive diagnosis require integrated cutoffs, gates or windows that permit scoring of samples as likely or not to have a condition. Instrument readers and software are often employed to collate data and process it through a diagnostic algorithm or decision tree.

For point of care diagnostics it is not feasible to directly monitor enzyme activities as this requires quite sophisticated assessment. Because most enzymes, including enzymes of interest are proteins, it is proposed that the amount of such enzymes can be detected by serological tests that detect the protein as an antigen ("mass concentration"), rather than by enzymatic activity. Most commonly, serological tests for detection of antigens will use a pair of binding agents and especially monoclonal antibodies produced in mice or other species to first bind, and secondly detect the antigen in question. A surprising and novel observation described herein is of a strong interference by human plasma on the level of antigenic reactivity with a range of mouse monoclonal antibodies. This led the inventor/s to conclude that interfering substance(s) in plasma bind to and obscure one or more of the antigenic epitopes in ALT1 that are able to induce an immune response in mice. It will be further appreciated that this general method can be applied to the development of test assays and especially POC test assays against other enzymes, including but not limited to other liver-specific enzymes that may be used for different purposes. For example, aspartate aminotransferase (AST) may be used instead of ALT for measurement of liver disease.

The present inventor/s reasoned that one or more factors present in human plasma may interfere with the binding of murine antibody to ALT and other antigens, thus preventing the development of useful sensitive assays for ALT mass concentration using this approach.

The surprising and novel observation of the strong interference by human plasma on the level of ALT antigenic reactivity with a range of mouse MAb led the inventor/s to conclude that interfering substance(s) in plasma may bind to and obscure one or more of the antigenic epitopes in ALT1 that are able to induce an immune response in mice.

The inventor/s devised a solution to this problem by taking advantage of the known fact that rabbits do not have a homolog of the human ALT1 protein or gene, and instead have only a homolog of the ALT2 protein that appears to provide the relevant biochemical functions in all forms of body tissues. Thus, all parts of the human ALT1 protein (and especially the surface part of the protein) may be expected to be immunogenic in rabbits, in contrast to the situation in mice where only the regions of difference between mouse and human ALT1 will be immunogenic. As shown in Figure 4, rabbits produced robust antibody responses after immunization with purified human ALT1. In one embodiment, an ELISA was established using rabbit anti-ALT to capture ALT in human plasma, and biotin-labelled rabbit anti-ALT and horseradish peroxidase-labelled streptavidin to detect the captured ALT. As shown in Figure 5, this ELISA was not adversely affected by the presence of human serum/plasma, making it suitable for the analytical determination of ALT mass concentration in human clinical specimens. Due to the sensitivity of the test, it would now be possible to detect diagnostically useful levels of ALT1 in subjects. To enable detection of ALT1 at the point of care, Figure 6 illustrates the detection method for ALT1 using colloidal gold as an alternative detection system to ELISA. Using this colloidal gold test format, it was shown that it is possible to detect ALT in human plasma or whole blood in the format of a lateral-flow, immunochromatographic test strip (Figure 7).

While the use of an instrument such as the AX-2 instrument in combination with the POC ALT test is desirable for many reasons, there is also a need for a test that can give a simple, visual readout of "normal" (healthy) versus "abnormal" (elevated, unhealthy) levels of ALT, for example < 40 IU/L, or ≥ 40 IU/L respectively according to EASL guidelines. This is readily achieved using methods previously described in development of a lateral flow, POC test for measuring CD4 T-cells (US Patent 8,409,818 and other territories) (Figure 9). This provides the additional advantage that similar instruments, test cartridges and manufacturing methods can be used for the various test devices.

The assays and kits are contemplated that employ one or more of flow cytometric quantification techniques (eg. fluorescent microbeads), microfluidic, cartridge, IFA, ELISA-type, and lateral flow devices, etc optionally together with an instrument reader and/or associated software to evaluate and compare ALT1 and or platelet levels.

It will be appreciated by one skilled in the art that this test can be further modified to provide for simultaneous measurement of ALT and one or more other relevant biomarkers, for example ALT plus platelet levels which are both important markers in management of pre-eclampsia.

It will be further appreciated that this general method can be applied to the development of test assays and especially POC test assays against other enzymes, including but not limited to other liver-specific enzymes that may be used for different purposes. For example, aspartate aminotransferase (AST) may be used instead of ALT for measurement of liver disease.

In addition, the combination of ALT plus platelet levels may provide a useful noninvasive biomarker for the extent of liver fibrosis, by analogy with enzymatic AST and platelet levels where the AST-platelet ratio index (APRI) score is the arithmetic product of the sample result for AST (divided by normal level), multiplied by 100 and divided by the platelet count (in 10⁹ per ml). APRI score is recommended by the World Health Organisation for the noninvasive measurement of liver fibrosis where there is not access to alternative methods such as Fibroscan (specialised ultrasound). Similarly, a POC test for APRI would combine the measurement of AST levels as described herein for ALT, and platelet levels.

While the ALT assays described herein have made use of affinity-purified polyclonal antibodies produced in rabbits, it will be understood that monoclonal antibodies or other antigen binding agents known in the art may be used. Techniques for the production of rabbit monoclonal antibodies are now available, and it is expected that rabbit MAbs against ALT1 could be readily obtained due to the lack of endogenous ALT1 in the rabbit. However in mice, the species most commonly used for production of MAbs, the presence of endogenous ALT1 results in most MAbs being directed against epitopes that may be susceptible to blocking by factor(s) present in human plasma, and this is likely to be true also in rats that also express ALT1. However the assays described herein for measurement of ALT1 and especially the ELISA assays allowed us to develop a novel assay for the screening and selection of antibodies and especially MAbs that are not susceptible to such plasma blocking and inhibition, and thus suitable for use in clinical assays such as the ALT assays described here.

As shown in Figure 11, when purified recombinant ALT is coated on an ELISA plate followed by sequential addition of human plasma (or no plasma as control) and a suitable dilution of the antibody or MAb of interest, the level of inhibition caused by addition of plasma can be determined, and antibody preparations or MAbs that are not substantially inhibited can be identified as a result. It will be appreciated by one skilled in the art that the same technique could be applied to antibodies produced in any animal species or by recombinant means, and also to non-antibody binding agents such as affimers, aptimers, peptide ligands identified by phage display and other methods, or nucleic acid binding ligands identified by various new methods.

Immunoassays can be done in any convenient format known in the art. These include Western blots, immunohistochemical assays and ELISA assays. The use of monoclonal antibodies in an immunoassay is widespread because of the ability to produce them in large quantities and the homogeneity of the product. Polyclonal antibodies are also widely used. The preparation of hybridoma cell lines for monoclonal antigen production is derived by fusing an immortal cell line and lymphocytes sensitized against the antigen of interest (in a non-limiting example the antibody is in the case of CD4 polypeptides, peptides 1 or 2 or homologs, derivatives, variants thereof) or can be done by techniques which are well known to those who are skilled in the art. (See, for example, Douillard and Hoffman, Basic Facts about Hybridomas, in Compendium of Immunology Vol. II, ed. by Schwartz, 1981; Kohler and Milstein, Nature 256: 495-499, 1975; European Journal of Immunology 6: 511-519, 1976 or more recent references Sambrook, Molecular Cloning: A Laboratory Manual, 3rd Edition, CSHLP, CSH, NY, 2001).

The presence of complexes may be evaluated using ELISA-type procedures. A wide range of immunoassay techniques are available as can be seen by U.S. Pat. Nos. 4,016,043, 4,424,279 and 4,018,653. These include both single-site and two-site or "sandwich" assays of the non-competitive types, as well as in the traditional competitive binding assays.

Sandwich assays are among the most useful and commonly used assays. A number of variations of the sandwich assay technique exist, and all are intended to be encompassed herein. Briefly, in a typical forward assay, an antibody is immobilized on a solid or semi-solid substrate and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added (before or after a washing step) and incubated, allowing time sufficient for the formation of another complex of antibody-antigen-labelled antibody. Any unreacted material may be washed away, and the presence of the marker is determined by observation of a signal produced by the detectable marker (reporter molecule). The results may be qualitative or quantitative, by simple observation of the visible signal, or may be quantitated by comparing with a control sample containing known amounts of marker. Variations on the forward assay include a simultaneous assay, in which both sample and labelled antibody are added simultaneously to the bound antibody. These techniques are well known to those skilled in the art, including any minor variations as will be readily apparent. In accordance with the present invention, the sample is generally a biological sample comprising biological fluid, and is most conveniently a whole blood sample such as capillary or venous blood that may optionally be treated with an anticoagulant.

In a typical forward sandwich assay, a first antibody having specificity for a marker is either covalently or passively bound to a solid or semi-solid support. The support is typically glass or a polymer, the most commonly used polymers being nitrocellulose, cellulose, polyacrylamide, nylon, polystyrene, polyvinyl chloride, polypropylene or mixture or derivatives of these. The solid supports may be in the form of tubes, beads, discs or microplates, or any other surface suitable for conducting an immunoassay. The binding processes are well-known in the art and generally consist of cross-linking covalently binding or physically adsorbing the polymer-antibody complex to the solid surface which is then washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to about 37° C. including 25° C.) to allow binding of any subunit present in the antibody. Following the incubation period, the antibody subunit solid phase is washed and incubated with a second antibody specific for a portion of the antigen. The second antibody is linked to a detectable marker which is used to indicate the binding of the second antibody to the antigen.

An alternative method involves immobilizing the target molecules in the biological sample and then exposing the immobilized target to specific antibody which may or may not be labelled with a detectable marker. Depending on the amount of target and the strength of the signal from the detectable marker, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule. A significant improvement of the bead-based methods involves tagging each bead with a unique identifier tag, such as an oligonucleotide or electrophoretic tag, so as to facilitate identification of the amino acid sequence of each library member. These improved bead-based methods are described in International Publication No. WO 93/06121.

The method may be a liquid phase method. In one example of a liquid phase immunoassay (see for example U.S. Pat. No. 6,632,603) the sample is contacted with an agent capable of binding a marker and a detector agent comprising a visually detectable agent such as colloidal gold or silver labelled. The test sample is applied by flowing onto a defined zone of an insoluble porous support film having a pore size impassable to a complex formed between the marker and its target, and if present, with a binding substance and a detector substance, but passable to the binding substance and detector substance while remaining uncomplexed in the absence of the desired target. If the target is present in the test specimen, the detector substance binds with the target and the binding substance to form a visually inspectable complex on the surface of the porous support film. After application of the test sample to the porous support, the surface of the porous support is visually inspected for colour to determine the presence and quantity or the absence of the marker being assayed.

In another assay, magnetic antibodies that bind to markers are used to tag markers and a high T_{c} superconducting quantum interference device is used to measure the amount of free and bound antibody and hence the presence or level of CSAP. A liposome immunomigration, liquid-phase competition strip immunoassay is, for example, described in Glorio-Paulet et al J Agric Food Chem 48 (5):1678-1682, 2000.

General formats and protocols for the conduct of various formats of ELISA are disclosed in the art and are known to those of skill in the field of diagnostics. For example, reference may be made to Chapter 11 of Ausubel (Ed) Current Protocols in Molecular Biology, 5th Edition, John Wiley & Sons, Inc, NY, 2002. Rundström, G et al. describe lateral Flow immunoassay using Europium (III) Chelate Microparticles and Time-Resolved Fluorescence for eosinophils and neutrophils in Whole Blood. Clinical Chemistry 53, 342-348 (2007).

The term "level" or "levels" also encompasses ratios of level/s of biomarkers.

ECLIA, ELISA and Luminex LabMAP immunoassays are examples of suitable assays to detect levels of ALT1. In one example, a first specific binding agent is attached to a support surface and a second binding reagent/antibody comprising a detectable group binds to the first antigen binding agent. Examples of detectable-groups include, for example and without limitation: fluorochromes, enzymes, epitopes for binding a second binding reagent (for example, when the second binding reagent/antibody is a mouse antibody, which is detected by a fluorescently-labeled anti-mouse antibody), for example an antigen or a member of a binding pair, such as biotin and avidin. The surface may be a planar surface, such as in the case of a typical grid-type array (for example, but without limitation, 96-welI plates and planar microarrays) or a non-planar surface, as with coated bead array technologies, where each "species" of bead is labelled with, for example, a fluorochrome (such as the Luminex technology described in U. S. Patent Nos. 6,599, 331,6, 592,822 and 6,268, 222), or quantum dot technology (for example, as described in U. S. Patent No. 6,306. 610). Such assays may also be regarded as laboratory information management systems (LIMS):

In the bead-type immunoassays, the Luminex LabMAP system can be utilized. The LabMAP system incorporates polystyrene microspheres that are dyed internally with two spectrally distinct fluorochromes. Using precise ratios of these fluorochromes, an array is created consisting of different microsphere sets with specific spectral addresses. Each microsphere set can possess a different reactant on its surface. Because microsphere sets can be distinguished by their spectral addresses, they can be combined, allowing up to 100 different analytes to be measured simultaneously in a single reaction vessel. A third fluorochrome coupled to a reporter molecule quantifies the biomolecular interaction that has occurred at the microsphere surface. Microspheres are interrogated individually in a rapidly flowing fluid stream as they pass by two separate lasers in the Luminex analyzer. High-speed digital signal processing classifies the microsphere based on its spectral address and quantifies the reaction on the surface in a few seconds *per* sample.

The subject contemplated herein is generally a human subject and may also be referred to as a patient, individual or recipient. The human subject may be neonatal or an infant, child, adolescent, teenager, young adult, adult or elderly adult of male or female gender. Control subjects are often selected groups of subjects referred to as a population of subjects/patients. Test samples are generally from subjects suspected of having or being at risk of liver disease, for example, however samples may also be collected from subjects in individual or general population screening to exclude liver disease. Control samples may reflect different subgroups such as subjects with sever liver disease, hepatitis etc. Any subject group can be usefully employed as a control population including "unhealthy" subjects such as immunocompromised or aged populations.

The binding agent may conveniently be an antibody or an antigen-binding fragment thereof. Other suitable binding agents are known in the art and include antigen binding constructs such as affimers, aptamers. As described herein assays are provided for screening potential specific binding agents for their ability to bind ALT1 in the presence of human (or other species as appropriate) plasma to enable the method.

The term "binding agent" and like terms, refers to any compound, composition or molecule capable of specifically or substantially specifically (that is with limited cross-reactivity) binding to an epitope on the biomarker. The "binding agent" generally has a single specificity. Notwithstanding, binding agents having multiple specificities for two or more biomarkers are also contemplated herein. The binding agents (or ligands) are typically antibodies, such as monoclonal antibodies, or derivatives or analogs thereof, but also include, without limitation: Fv fragments; single chain Fv (scFv) fragments; Fab' fragments; F(ab')2 fragments; humanized antibodies and antibody fragments; camelized antibodies and antibody fragments, and multivalent versions of the foregoing. Multivalent binding reagents also may be used, as appropriate, including without limitation: monospecific or bispecific antibodies; such as disulfide stabilized Fv fragments, scFv tandems [(scFv) 2 fragments], diabodies, tribodies or tetrabodies, which typically are covalently linked or otherwise stabilized (i.e. leucine zipper or helix stabilized) scFv fragments. "Binding agents" also include aptamers, as are described in the art.

Methods of making antigen-specific binding agents, including antibodies and their derivatives and analogs and aptamers, are well-known in the art. Polyclonal antibodies can be generated by immunization of an animal. Monoclonal antibodies can be prepared according to standard (hybridoma) methodology. Antibody derivatives and analogs, including humanized antibodies can be prepared recombinantly by isolating a DNA fragment from DNA encoding a monoclonal antibody and subcloning the appropriate V regions into an appropriate expression vector according to standard methods. Phage display and aptamer technology is described in the literature and

A chromatographic device may be provided comprising material which has a pore size which allows or facilitates capillary flow of the components of the method. In some embodiments, the device comprises portions comprising material of different pore size, or nonporous material, the material being contiguous with the first material and designed to receive a sample or receive or store components of the method. The portions of the chromatographic device may be separate, contiguous or overlapping or designed to come together in use.

The sample pad may be chromatographically connected to a test portion of the device, the test portion comprising a binding agent such as an antibody or an antigen binding fragment thereof. The sample pad may be chromatographically connected to a test portion of the device, the test portion comprising a binding agent such as an antibody or an antigen binding fragment thereof.

When chromatographically active portions of the sample to be tested move from the sample pad towards and through the test portion, enzyme or liver enzyme or metabolites or ALT1 may be captured onto test or control portions (including strips) of the device and the remainder of the sample flowing from the sample pad is uncaptured. The uncaptured components of the test sample may be collected chromatographically into an absorbent pad, which is positioned in any orientation with respect to the test portion. Components of the subject sample, such as red blood cells or particular white blood cells may be retained in the sample pad, for example, by selecting a pad of suitable mesh or pore size and/or by the inclusion of specific reagents such as antibodies or lectins to bind and retain these components.

Once the test portion of the immunochromatographic device have been exposed to (say) ALT1 in the subject sample, the method proceeds by allowing contact between a/the test portions and a detection binding agent. The detection marker/s may be stored in a separate portion of the kit.

The detection marker comprises a visually detectable reporter molecule and a positive result may be essentially immediately observed in the test and/or control portions of the immunochromatographic device. The detection marker may be detected using further detection protocols and devices such as will be well known to those of ordinary skill in the art. For example, colloidal metal or metal oxide particles or colloidal non-metal particles or dyes or colored latex are conveniently used.

Numerous labels for binding agents such as antibodies or antigen binding fragments thereof are available which can be generally grouped into categories. The binding agent can be labelled with a radioisotope using the techniques described in Current Protocols in Immunology, Volumes 1 and 2, Coligen et al., Ed. Wiley-Interscience, New York, N.Y., Pubs. (1991), for example, and radioactivity can be measured using scintillation counting. Fluorescent labels may be used such as rare earth chelates (europium chelates) or fluorescein and its derivatives, rhodamine and its derivatives, dansyl, Lissamine, phycoerythrin and Texas Red are available. The fluorescent labels can be conjugated to the binding agent using the techniques disclosed in Current Protocols in Immunology (supra). Fluorescence can be quantified using a fluorimeter. Various enzyme-substrate labels are available and U.S. Pat. No. 4,275,149 provides a review of some of these. The enzyme generally catalyzes a chemical alteration of the chromogenic substrate that can be measured using various techniques. For example, the enzyme may catalyse a colour change in a substrate, which can be measured spectrophotometrically. Alternatively, the enzyme may alter the fluorescence or chemiluminescence of the substrate.

Techniques for quantifying a change in fluorescence are described above. The chemiluminescent substrate becomes electronically excited by a chemical reaction and may then emit light that can be measured (using a chemiluminometer, for example) or donates energy to a fluorescent acceptor. Examples of enzymatic labels include luciferases (e.g., firefly luciferase and bacterial luciferase; US Patent No. 4,737,456), luciferin, 2,3-dihydrophthalazinediones, malate dehydrogenase, urease, peroxidase such as horseradish peroxidase (HRPO), alkaline phosphatase, .beta.-galactosidase, glucoamylase, lysozyme, saccharide oxidases (eg, glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed J. Langone and H. Van Vunakis), Academic press, New York, 73:147-166 (1981).

Examples of enzyme-substrate combinations include, for example: Horseradish peroxidase (HRPO) utilizes hydrogen peroxide to oxidize a dye precursor (e.g., orthophenylene diamine (OPD) or 3,3',5,5'-tetramethyl benzidine hydrochloride (TMB));] alkaline phosphatase (AP) with para-Nitrophenyl phosphate as chromogenic substrate; and 3) β-D-galactosidase with a chromogenic substrate (e.g., p-nitrophenyl-.beta.-D-galactosidase) or fluorogenic substrate 4-methylumbelliferyl-.beta.-D-galactosidase. Numerous other enzyme-substrate combinations are available to those skilled in the art. For a general review of these, see US Patent Nos. 4,275,149 and 4,318,980.

Sometimes, the label is indirectly conjugated with the antibody. The skilled artisan will be aware of various techniques for achieving this. For example, the antibody can be conjugated with biotin and any of the three broad categories of labels mentioned above can be conjugated with avidin or streptavidin, or vice versa. Biotin binds selectively to avidin and thus, the label can be conjugated with the antibody in this indirect manner. Alternatively, to achieve indirect conjugation of the label with the antibody, the antibody is conjugated with a small hapten (e.g., digoxin or biotin) and one of the different types of labels mentioned above is conjugated with an anti-hapten antibody (e.g., anti-digoxin antibody or anti-biotin antibody). Thus, indirect conjugation of the label with the antibody can be achieved. The antibody need not be labeled, and the presence thereof can be detected using a labeled antibody which binds to the antibody. The binding agent may be employed in any known assay method, such as competitive binding assays, direct and indirect sandwich assays, and immunoprecipitation assays. Zola, Monoclonal Antibodies: A Manual of Techniques, pp. 47-158 (CRC Press, Inc. 2011).

For use in the applications described or suggested herein, diagnostic assay kits or articles of manufacture are also provided. Such kits may comprise a carrier means being compartmentalized to receive in close confinement one or more container means such as vials, tubes, and the like, each of the container means comprising one of the separate elements to be used in the method. For example, one of the container means may reporter means, such as a biotin-binding protein, such as avidin or streptavidin, bound to a reporter molecule, such as an enzymatic, florescent, or radioisotope label.

Diagnostic assay kits will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific therapy or non-therapeutic application, and may also indicate directions for use, such as those described above. Other optional components in the kit include one or more buffers (e.g., block buffer, wash buffer, substrate buffer, and the like), other reagents such as substrate (e.g., chromogen) which is chemically altered by an enzymatic label, epitope retrieval solution, control samples (positive and/or negative controls), control slide(s) etc.

The terms "full-length antibody," or "whole antibody" are used interchangeably to refer to an antibody in its substantially intact form, as opposed to an antigen binding fragment of an antibody. Specifically, whole antibodies include those with heavy and light chains including a constant region. The constant region may be wild-type sequence constant regions or amino acid sequence variants thereof.

The term "antigen binding protein" herein includes a single polypeptide chain, (i.e., a series of contiguous amino acids linked by peptide bonds), or a series of polypeptide chains covalently or non-covalently linked to one another (i.e., a polypeptide complex) capable of binding to (say) ALT1 in the manner described and/or claimed herein. For example, the series of polypeptide chains can be covalently linked using a suitable chemical or a disulphide bond. Examples of non-covalent bonds include hydrogen bonds, ionic bonds, Van der Waals forces, and hydrophobic interactions. A non-covalent bond contemplated by the present disclosure is the interaction between a VH and a VL, e.g., in some forms of diabody or a triabody or a tetrabody or an Fv.

As used herein, "variable region" refers to the portions of the light and/or heavy chains of an antibody or of a heavy chain only antibody (e.g., camelid antibodies or cartilaginous fish immunoglobulin new antigen receptors (IgNARs)) that is capable of specifically binding to an antigen and includes amino acid sequences of complementary determining regions "CDRs"; i.e., CDRl, CDR2, and CDR3, and FRs. For example, the variable region comprises three or four FRs (e.g., FR1, FR2, FR3 and optionally FR4) together with three CDRs. VH refers to the variable region of the heavy chain. VL refers to the variable region of the light chain.

As used herein, the term "Fv" shall be taken to mean any protein, whether comprised of multiple polypeptides or a single polypeptide, in which a VL and a VH associate and form a complex having an antigen binding domain, i.e., capable of specifically binding to an antigen (e.g.,ALT1). The VH and the VL which form the antigen binding domain can be in a single polypeptide chain or in different polypeptide chains. This term shall be understood to encompass fragments directly derived from an antibody as well as proteins produced using recombinant means. In some examples, the VH is not linked to a heavy chain constant domain CH1 and/or the VL is not linked to a light chain constant domain (CL), e.g., a domain antibody. Exemplary Fv containing polypeptides or proteins include a Fab fragment, a Fab' fragment, a F(ab') fragment, a scFv, a diabody, a triabody, a tetrabody or higher order complex, or any of the foregoing linked to a constant region or domain thereof, e.g., CH2 or CH3 domain, e.g., a minibody.

A "Fab fragment" consists of a monovalent antigen-binding fragment of an immunoglobulin, and can be produced by digestion of a whole antibody with the enzyme papain, to yield a fragment consisting of an intact light chain and a portion of a heavy chain or can be produced using recombinant means. A "Fab' fragment" of an antibody can be obtained by treating a whole antibody with pepsin, followed by reduction, to yield a molecule consisting of an intact light chain and a portion of a heavy chain comprising a VH and a single constant domain. Two Fab' fragments are obtained per antibody treated in this manner. A Fab' fragment can also be produced by recombinant means. An "F(ab')2 fragment" of an antibody consists of a dimer of two Fab' fragments held together by two disulfide bonds, and is obtained by treating a whole antibody molecule with the enzyme pepsin, without subsequent reduction. An "Fab2" fragment is a recombinant fragment comprising two Fab fragments linked using, for example a leucine zipper or a CH3 domain. A "single chain Fv" or "scFv" is a recombinant molecule containing the variable region fragment (Fv) of an antibody in which the variable region of the light chain and the variable region of the heavy chain are covalently linked by a suitable, flexible polypeptide linker.

As used herein, the term "antigen binding domain" shall be taken to mean a region of an antibody that is capable of specifically binding to an antigen, i.e., a VH or a VL or a Fv or a variable region as defined herein. The antigen binding domain need not be in the context of an entire antibody, e.g., it can be in isolation (e.g., a domain antibody) or in another form, such as a scFv.

As used herein, the term "binds" in reference to the interaction of a protein or an antigen binding domain thereof with an antigen means that the interaction is dependent upon the presence of a particular structure (e.g., an antigenic determinant or epitope) on the antigen. For example, an antibody recognizes and binds to a specific protein structure rather than to proteins generally. If an antibody binds to epitope "A", the presence of a molecule containing epitope "A" (or free, unlabeled "A"), in a reaction containing labeled "A" and the antibody, will reduce the amount of labeled "A" bound to the antibody.

As used herein, the term "specifically binds" or "specific binding agent" or "specific antibody" means a binding agent reacts or associates more frequently, more rapidly, with greater duration and/or with greater affinity with a particular antigen than it does with alternative antigens. For example, a protein that specifically binds to an antigen binds that antigen with greater affinity (e.g., 20 fold or 40 fold or 60 fold or 80 fold to 100 fold or 150 fold or 200 fold greater affinity), avidity, more readily, and/or with greater duration than it binds to other antigens, e.g., to ALT2. The binding agent may selectively bind the antigen which means that binding is exclusive to the specified antigen.

The description includes the following non-limiting examples to illustrate the invention.

### Example 1 -Human plasma interferes strongly with the ability of a range of mouse antibodies to bind ALT

Commercially-available ELISAs for measurement of ALT in human plasma also exhibit very poor correlation with enzymatic ALT levels, and surprisingly yield results that are highly dependent on the sample dilution used for serum or plasma (see Figure 1).

As shown in Figure 2, initial experiments disclosed herein by the inventor/s provided similar results. Using a range of MAbs produced in mice using the ALT1 protein as immunogen (Figure 2), the results were highly dependent on the sample dilution used for serum or plasma. However, the present inventor/s reasoned that one or more factors present in human plasma may interfere with the antigenic reactivity of ALT with antibody, and thus preventing the development of useful assays for ALT mass concentration using this approach.

The surprising and novel observation of the strong interference by human plasma on the level of ALT antigenic reactivity with a range of mouse MAb led the inventor/s to conclude that interfering substance(s) in plasma may bind to and obscure one or more of the antigenic epitopes in ALT1 that are able to induce an immune response in mice.

### Example 2- Rabbits produce robust antibodies against human ALT1

The inventor/s compared the amino acid sequence of ALT1 in humans and in mice (see Figure 3A) and identified the proteins have high levels of identity. Accordingly, it may be expected that only certain parts of the protein surface are sufficiently different between humans (the antigen) and mice (the host animal) to be able to function as foreign antigens to induce an immune response. Furthermore, the limited number of highly antigenic sites may overlap with the sites that bind factor(s) present in human plasma, preventing proper recognition of the ALT protein when assayed in human plasma using the antibodies directed against such sites.

The inventor/s devised a solution to this problem by taking advantage of the known fact that rabbits do not have a homolog of the human ALT1 protein or gene, and instead have only a homolog of the ALT2 protein that appears to provide the relevant biochemical functions in all forms of body tissues. Thus, substantially all parts of the human ALT1 protein (and especially the surface part of the protein) may be expected to be immunogenic in rabbits, in contrast to the situation in mice where only the limited regions of difference between mouse and human ALT1 will be immunogenic.

As shown in Figure 4, rabbits produced robust antibody responses after immunization with purified human ALT1.

### Example 3 -Lack of interference by human plasma in the results of Rabbit antibody ELISA

After purification of specific anti-ALT antibodies from the total rabbit IgG by antigenaffinity chromatography, an ELISA was established using rabbit anti-ALT to capture ALT in human plasma, and biotin-labelled rabbit anti-ALT and horseradish peroxidase-labelled streptavidin to detect the captured ALT.

As shown in Figure 5, this ELISA was not adversely affected by the presence of human serum/plasma, making it suitable for the analytical determination of ALT mass concentration in human clinical specimens. Figure 6 illustrates the strategy used to convert the ELISA format of the test into a colloidal gold detection method suitable for lateral flow immunochromatography.

### Example 4 - Detection of ALT in human plasma or whole blood in the format of a lateral-flow, immunochromatographic test strip

Using further samples of the same affinity-purified rabbit anti-ALT and the strategy illustrated in Figure 6, it was shown that it is possible to detect ALT in human plasma or whole blood in the format of a lateral-flow, immunochromatographic test strip (Figure 7).

In these examples, it is readily observed that the enzymatic level of ALT in the clinical specimens shows a visual correlation with the intensity of the ALT test line in the POC test strips. Measurement of the ALT test line intensity using the AX-2 instrument (Figure 10) provides a numerical value for each test and allows the correlation between test line intensity (peak height) and enzymatic ALT values to be determined (Figure 8A and 8B), showing close correlation between the two values. This is in contrast to a lateral-flow, immunochromatographic test for ALT1 using mouse monoclonal antibodies described by Hu et al (2016), where there was very low correlation with enzymatic ALT levels, presumably because of variable levels of interference by plasma proteins as we have noted herein.

Interestingly, in our example a small number of samples demonstrate substantially elevated amounts of ALT mass concentration versus enzymatic ALT when compared to the average level of correlation (line of best fit), which may represent patients with more advanced forms of liver disease and having elevated ALT mass concentration compared to enzymatic ALT, as reported by Kim et al (2006). Therefore the ALT test described here provides the benefit of strong correlation with standard enzymatic ALT tests (in contrast to Hu et al [2016]), but with the additional capacity to detect elevated ALT mass concentration in patients with advanced liver disease where ALT production or activity may be compromised giving a false indication of normal (healthy) levels of ALT when measured by enzymatic activity.

### Example 5 -Visual readout of "normal" (healthy) versus "abnormal" (elevated, unhealthy) levels of ALT

While the use of an instrument such as the AX-2 instrument in combination with the POC ALT test is desirable for many reasons, there is also a need for a test that can give a simple, visual readout of "normal" (healthy) versus "abnormal" (elevated, unhealthy) levels of ALT, for example < 40 IU/L, or ≥ 40 IU/L respectively according to EASL guidelines. This is readily achieved using methods previously described in development of a lateral flow, POC test for measuring CD4 T-cells (US Patent 8,409,818 and other territories) (Figure 9). This provides the additional advantage that similar instruments, test cartridges and manufacturing methods can be used for the various test devices.

In the CD4 T-cell test, the amount of CD4 antigen represented at the test (T) line is directly proportional to the number of CD4 T-cells. The test signal is then compared visually or by instrument reader versus one or more reference lines representing clinically relevant levels of CD4 T-cells, in the figure the example of 350 T-cells per µl which is recommended by WHO for prioritisation of antiretroviral therapy in HIV infection. Similarly, for ALT we will prepare a reference line corresponding to one or more clinically relevant amounts of ALT, including the 40 IU/L level recommended by EASL. A schematic of the proposed semi-quantitative ALT test is shown in Figure 10.

It will be appreciated by one skilled in the art that this test can be further modified to provide for simultaneous measurement of ALT and one or more other relevant biomarkers, for example ALT plus platelet levels which are both important markers in management of pre-eclampsia.

It will be further appreciated that this general method can be applied to the development of test assays and especially POC test assays against other enzymes, including but not limited to other liver-specific enzymes that may be used for different purposes. For example, aspartate aminotransferase (AST) may be used instead of ALT for measurement of liver disease.

In addition, the combination of ALT plus platelet levels may provide a useful noninvasive biomarker for the extent of liver fibrosis, by analogy with enzymatic AST and platelet levels where the AST-platelet ratio index (APRI) score is the arithmetic product of the sample result for AST (divided by normal level), multiplied by 100 and divided by the platelet count (in 10⁹ per ml). APRI score is recommended by the World Health Organisation for the noninvasive measurement of liver fibrosis where there is not access to alternative methods such as Fibroscan (specialised ultrasound). Similarly, a POC test for APRI would combine the measurement of AST levels as described herein for ALT, and platelet levels.

### Example 6 -Screening for agents that bind ALT or other enzymes

While the ALT assays described herein have made use of affinity-purified polyclonal antibodies produced in rabbits, it will be understood that monoclonal antibodies or other binding agents may be preferred for this purpose. Techniques for the production of rabbit monoclonal antibodies are now available, and it is expected that rabbit MAbs against ALT1 could be readily obtained due to the lack of endogenous ALT1 in the rabbit. However in mice, the species most commonly used for production of MAbs, the presence of endogenous ALT1 results in most MAbs being directed against epitopes that may be susceptible to blocking by factor(s) present in human plasma, and this is likely to be true also in rats that also express ALT1 very similar to human (Figure 3B). However the assays described herein for measurement of ALT1 and especially the ELISA assays allowed us to develop a novel assay for the screening and selection of antibodies and especially MAbs that are not susceptible to such plasma blocking and inhibition, and thus suitable for use in clinical assays such as the ALT assays described here.

As shown in Figure 11A, when purified recombinant ALT is coated on an ELISA plate followed by sequential addition of human plasma (or no plasma as control) and a suitable dilution of the antibody or MAb of interest, the level of inhibition caused by addition of plasma can be determined, and antibody preparations or MAbs that are not substantially inhibited can be identified as a result. It will be appreciated by one skilled in the art that the same technique could be applied to antibodies produced in any animal species or by recombinant means, and also to non-antibody binding agents such as peptide ligands identified by phage display and other methods, or nucleic acid binding ligands (aptamers) identified by various methods.

### Example 7 -Specificity of the assay for the liver-specific ALT1 isoform

As described above, in humans the ALT1 isoform is predominantly expressed in the liver whereas the ALT2 isoform is predominantly expressed in muscle and other tissues, thus an assay that is specific for ALT1 versus ALT2 is expected to have more clinical utility for detection and monitoring of liver disease.

As shown in Figure 12, when unpurified human ALT1 or human ALT2 (Seq ID: 6) representing equivalent levels of enzymatic ALT activity are coated on ELISA plates followed by rabbit antibodies against ALT1, ELISA reactivity is only seen for ALT1 and not ALT2 for all concentrations of enzymatic ALT, demonstrating specificity of the reagent and the assay for ALT1 and not ALT2.

Similarly in Figure 13, when unpurified human ALT1 or human ALT2 (Seq ID: 6) representing equivalent levels of enzymatic ALT activity are subjected to the immunochromatographic assay as in Figure 7 (Example 4), only ALT1 is detected in the immunochromatographic test.

### BIBLIOGRAPHY

Younossi ZM, Koenig AB, Abdelatif D, Fazel Y, Henry L, Wymer M. 2016._Global epidemiology of nonalcoholic fatty liver disease - Meta-analytic assessment of prevalence, incidence, and outcomes. Hepatology 64(1):73-84.
European Association for the Study of the Liver. 2012. EASL Clinical Practice Guidelines: management of chronic hepatitis B virus infection. J Hepatol. 57:167--185.
Pollock NR, Rolland JP, Kumar S, Beattie PD, Jain S, Noubary F, Wong VL, Pohlmann RA, Ryan US, Whitesides GM. 2012. A Paper-Based Multiplexed Transaminase Test for Low-Cost, Point-of-Care Liver Function Testing. Science Translational Medicine 4, 152ra129.
Kim HJ, Oh SW, Kim DJ, Choi EY. 2009. Abundance of Immunologically Active Alanine Aminotransferase in Sera of Liver Cirrhosis and Hepatocellular Carcinoma Patients. Clinical Chemistry 55:5, 1022-1025.
Hu X, Cheng S, Liu X, Li J, Zheng W, Lu G, Zhang J, Zheng J, Zhang J. 2015. Development of monoclonal antibodies and immunochromatographic lateral flow device for rapid test of alanine aminotransferase isoenzyme 1. Protein Expression and Purification 119:94-101.
Hassan et.al. A microfluidic biochip for complete blood cell counts at the point of care. Technology 3, 201-213 (2015).19.

## Claims

1. An in vitro immunoassay suitable for point of care to assess liver disease or function, said assay comprising:
i. contacting a blood sample from a human subject with a specific binding agent that recognizes an epitope of ALT1, that is not recognized by rodent antibodies raised against human ALT1 when the rodent antibodies are in the presence of human plasma, to form an antigen-binding agent complex and detecting the complex using a second or further binding agent linked to or comprising a detectable reporter; and
ii. detecting liver disease or liver function in the subject contingent upon the mass concentration of ALT1 in the sample determined from step (i);
wherein the specific binding agent is a lagomorph antibody or comprises an antigen binding part thereof, or wherein the specific binding agent is an antibody or antigen binding part thereof that recognizes the same epitope as the lagomorph antibody.

2. The assay of claim 1, wherein:
(a) the epitope of ALT1 is not immunodominant in rodents; and/or
(b) the mass concentration of the ALT1 is positively correlated with ALT1 enzymatic activity;
and/or
(c) the mass concentration of ALT1 is positively correlated with increasing liver disease; and/or
(d) the immunoassay detects a mass concentration of human ALT1 that is equivalent to the upper limit of normal human ALT1 enzyme activity.

3. The assay of claims 1 or 2, wherein the assay is an enzyme-linked immunosorbent (ELISA)-type or immunochromatographic-type assay or microfluidic assay; and optionally wherein the binding agent is immobilised on a support.

4. The assay of claim 3, wherein the assay is an immunochromatographic-type assay and wherein the blood sample is contacted with the binding agent by applying the blood sample to a sample portion of an immunochromatographic device wherein the device sample portion is operably connected to a spaced capture portion of the device and whereby the components of the sample flow from the device sample portion and wherein one capture portion comprises the binding agent which specifically binds to the antigen in the sample such that the antigen is captured by the binding agent to form a binding agent-antigen complex in the capture portion; optionally wherein the capture portion is a test line.

5. The assay of any one of claims 1 to 4 wherein the antigen complex is detected using a binding agent such as an antibody or antigen-binding fragment, ligand or affimer that specifically binds the antigen and directly or indirectly provide a detectable signal that can be quantified visually or by instrument reader.

6. A kit for measuring the mass concentration of ALT1 in a blood sample from a human subject comprising (i) a chromatographic device comprising a porous membrane, or a microfluidic device, operably connected to a sample portion, one or more capture (test) portions, and optionally one or more of the following; a conjugate (detection marker) portion, a sucker component, a suitable control portion and optionally a cell lysis or solubilisation portion, and (ii) a lagomorph antibody or an agent comprising the antigen binding part thereof that recognizes an epitope of human ALT1 that is not recognized by rodent antibodies raised against human ALT1, and forms an ALT1-lagomorph antibody/binding part complex, and (iii) optionally instructions for using the device to determine liver function; optionally wherein the capture portion is a test line.

7. An *in vitro* method of screening for antibodies that are substantially not inhibited from ALT1 binding by human plasma, the method comprising contacting human ALT1 and a potential antibody in the presence of different concentrations of human plasma to determine whether the antibody can bind the antigen in the presence of plasma, wherein the antibody is a lagomorph antibody or comprises an antigen binding part thereof.

8. The assay of any one of claims 1 to 5 further comprising detecting platelet levels and determining liver function in the subject contingent upon the mass concentration of liver enzyme and platelet levels in the sample .

9. The kit of claim 6 further comprising reagents for assessing platelet levels.

10. A kit comprising (i) a chromatographic device comprising a porous membrane or a microfluidic device operably connected to a sample portion, two or more capture (test) portions, and optionally one or more of the following; a conjugate (detection marker) portion, a sucker portion, a suitable control portion and optionally a cell lysis or solubilisation portion, and (ii) a specific binding agent that recognizes an epitope of ALT1 or other liver enzyme or metabolite thereof, that is not recognized by or in competition with rodent antibodies when the liver enzyme or metabolite are in the presence of human plasma, and forms a liver enzyme-antibody/binding agent complex, and a second binding agent that binds specifically to platelets in the sample and forms a platelet marker-binding agent complex wherein the binding agents are either immobilised to separate capture portions and/or contained within conjugate portions and (iii) optionally instructions for using the device to determine the liver enzyme-platelet ratio index as a measure of liver function/fibrosis and wherein the specific binding agent is a lagomorph antibody or comprises an antigen binding part thereof, or wherein the specific binding agent recognizes the same epitope as the lagomorph antibody.

## Patentansprüche

1. In-vitro-Immuntest, der für die Verwendung am Einsatzort zur Beurteilung von Lebererkrankungen oder -funktion geeignet ist, wobei der Test folgendes umfasst:
i. In-Kontakt-Bringen einer Blutprobe eines menschlichen Probanden mit einem spezifischen Bindemittel, das ein Epitop von ALT1 erkennt, das von Nagetier-Antikörpern, die gegen menschliches ALT1 erzeugt wurden, nicht erkannt wird, wenn sich die Nagetier-Antikörper in Anwesenheit von menschlichem Plasma befinden, um einen Antigen-Bindemittel-Komplex auszubilden, und Detektieren des Komplexes unter Verwendung eines zweiten oder weiteren Bindemittels, das mit einem detektierbaren Reporter verbunden ist oder diesen umfasst; und
ii. Detektieren einer Lebererkrankung oder einer Leberfunktion bei dem Probanden in Abhängigkeit von der in Schritt (i) bestimmten Massenkonzentration von ALT1 in der Probe;
wobei das spezifische Bindemittel ein Kaninchen-Antikörper ist oder einen Antigen-Bindungsteil davon umfasst, oder wobei das spezifische Bindemittel ein Antikörper oder ein Antigen-Bindungsteil davon ist, der das gleiche Epitop wie der Kaninchen-Antikörper erkennt.

2. Test nach Anspruch 1, wobei:
(a) das Epitop von ALT1 bei Nagetieren nicht immundominant ist; und/oder
(b) die Massenkonzentration des ALT1 positiv mit der ALT1-Enzymaktivität korreliert ist; und/oder
(c) die Massenkonzentration von ALT1 positiv mit einer fortschreitenden Lebererkrankung korreliert ist; und/oder
(d) der Immuntest eine Massenkonzentration von menschlichem ALT1 detektiert, die der Obergrenze der normalen menschlichen ALT1-Enzymaktivität entspricht.

3. Test nach Anspruch 1 oder 2, wobei der Test ein enzymgebundener Immunosorbent, (ELISA)-Typ oder ein immunochromatographischer Typ oder ein mikrofluidischer Test ist; und/oder wobei das Bindemittel optional auf einem Träger immobilisiert ist.

4. Test nach Anspruch 3, wobei der Test ein immunchromatographischer Typ ist und wobei die Blutprobe mit dem Bindemittel in Kontakt gebracht wird, indem die Blutprobe auf einen Probenabschnitt einer immunchromatographischen Vorrichtung aufgebracht wird, wobei der Probenabschnitt der Vorrichtung funktionsfähig mit einem beabstandeten Einfangbereich der Vorrichtung verbunden ist und wobei die Komponenten der Probe aus dem Probenabschnitt der Vorrichtung fließen und wobei ein Einfangbereich das Bindemittel umfasst, das spezifisch an das Antigen in der Probe bindet, derart, dass das Antigen durch das Bindemittel eingefangen wird, um einen Bindemittel-Antigen-Komplex in dem Einfangbereich auszubilden; wobei der Einfangbereich optional eine Testlinie ist.

5. Test nach einem der Ansprüche 1 bis 4, wobei der Antigen-Komplex unter Verwendung eines Bindemittels wie eines solchen Antikörpers oder eines antigenbindenden Fragments, Liganden oder Affimers detektiert wird, die spezifisch das Antigen binden und direkt oder indirekt ein detektierbares Signal bereitstellen, das visuell oder mit einem Lesegerät quantifiziert werden kann.

6. Set zur Messung der Massenkonzentration von ALT1 in einer Blutprobe eines menschlichen Probanden, umfassend (i) eine chromatographische Vorrichtung, umfassend eine poröse Membrane oder eine mikrofluidische Vorrichtung, die funktionsfähig mit einem Probenabschnitt, einem oder mehreren Einfang-(Test-) Bereichen und optional einem oder mehreren der Folgenden verbunden ist; einen Konjugatteil (Detektionsmarker), eine Saugkomponente, einen geeigneten Kontrollteil und optional einen Zelllyse- oder Solubilisierungsteil, und (ii) einen Kaninchen-Antikörper oder ein Mittel, das den Antigen-Bindungsteil davon umfasst, der ein Epitop von menschlichem ALT1 erkennt, das von Nagetier-Antikörpern, die gegen menschliches ALT1 erzeugt wurden, nicht erkannt wird, und einen ALT1-Kaninchen-Antikörper- /Bindungsteil-Komplex ausbildet, und (iii) optional Anweisungen zur Verwendung der Vorrichtung zur Bestimmung der Leberfunktion; optional, wobei der Einfangbereich eine Testlinie ist.

7. *In-vitro-Verfahren* zur Überprüfung von Antikörpern, die wesentlich nicht an der Bindung von ALT1 durch menschliches Plasma gehindert werden, wobei das Verfahren das In-Kontakt-bringen von menschlichem ALT1 und einem potentiellen Antikörper in Anwesenheit unterschiedlicher Konzentrationen von menschlichem Plasma umfasst, um zu bestimmen, ob der Antikörper das Antigen in Anwesenheit von Plasma binden kann, wobei der Antikörper ein Kaninchen-Antikörper ist oder einen Antigen-Bindungsteil davon umfasst.

8. Test nach einem der Ansprüche 1 bis 5, ferner umfassend das Detektieren von Blutplättchenwerten und Bestimmen der Leberfunktion des menschlichen Probanden in Abhängigkeit von der Massenkonzentration des Leberenzyms und der Blutplättchenwerte in der Probe.

9. Set nach Anspruch 6, ferner umfassend Reagenzien zum Bestimmen der Blutplättchenwerte.

10. Set, umfassend (i) eine chromatographische Vorrichtung, umfassend eine poröse Membrane oder eine mikrofluidische Vorrichtung, die funktionsfähig mit einem Probenabschnitt, zwei oder mehreren Einfang-(Test-)Bereichen verbunden ist, und optional einen oder mehrere der Folgenden; einen Konjugatteil(Detektionsmarker), eine Saugkomponente, einen geeigneten Kontrollteil und optional einen Zelllyse- oder Solubilisierungsteil, und (ii) ein spezifisches Bindemittel, das ein Epitop von ALT1 oder eines anderen Leberenzyms oder eines Metaboliten davon erkennt, das nicht von oder in Konkurrenz mit Nagetier-Antikörpern erkannt wird, wenn sich das Leberenzym oder der Metabolit in Anwesenheit von menschlichem Plasma befinden und einen Leberenzym-Antikörper-Bindemittel-Komplex ausbildet und ein zweites Bindemittel, das sich spezifisch an Blutplättchen in der Probe bindet und einen Blutplättchenmarker-Bindemittel-Komplex bildet, wobei die Bindemittel entweder an separate Einfangbereiche immobilisiert und/oder in Konjugatteilen enthalten sind und (iii) optional Anweisungen zur Verwendung der Vorrichtung zur Bestimmung des Leberenzym-Blutplättchen-Verhältnis-Index als Maß für die Leberfunktion/Fibrose und wobei das spezifische Bindemittel ein Kaninchen-Antikörper ist oder einen Antigen-bindenden Teil davon umfasst oder wobei das spezifische Bindemittel dasselbe Epitop wie der Kaninchen-Antikörper erkennt.

## Revendications

1. Dosage immunologique in vitro adapté au point d'intervention pour évaluer une maladie ou une fonction hépatique, ledit dosage comprenant :
i. la mise en contact d'un échantillon de sang d'un sujet humain avec un agent de liaison spécifique qui reconnaît un épitope d'ALT1, qui n'est pas reconnu par des anticorps de rongeur dirigés contre l'ALT1 humaine lorsque les anticorps de rongeur sont en présence de plasma humain, pour former un complexe antigène-agent de liaison et détecter le complexe à l'aide d'un second ou d'un autre agent de liaison lié à ou comprenant un rapporteur détectable ; et
ii. la détection d'une maladie hépatique ou une fonction hépatique chez le sujet en fonction de la concentration massique d'ALT1 dans l'échantillon déterminé à l'étape (i) ;
dans lequel l'agent de liaison spécifique est un anticorps lagomorphe ou comprend une partie de liaison à l'antigène de celui-ci, ou dans lequel l'agent de liaison spécifique est un anticorps ou une partie de liaison à l'antigène de celui-ci qui reconnaît le même épitope que l'anticorps lagomorphe.

2. Dosage selon la revendication 1, dans lequel :
(a) l'épitope d'ALT1 n'est pas immunodominant chez les rongeurs ; et/ou
(b) la concentration massique de l'ALT1 est positivement corrélée à l'activité enzymatique de l'ALT1 ; et/ou
(c) la concentration massique d'ALT1 est positivement corrélée à l'augmentation des maladies hépatiques ; et/ou
(d) le dosage immunologique détecte une concentration massique d'ALT1 humaine équivalente à la limite supérieure de l'activité enzymatique ALT1 humaine normale.

3. Dosage selon les revendications 1 ou 2, dans lequel le dosage est un dosage de type immuno-absorption enzymatique (ELISA) ou de type immunochromatographique ou un dosage microfluidique ; et éventuellement dans lequel l'agent de liaison est immobilisé sur un support.

4. Dosage selon la revendication 3, dans lequel le dosage est un dosage de type immunochromatographique et dans lequel l'échantillon de sang est mis en contact avec l'agent de liaison par application de l'échantillon de sang sur une partie d'échantillon d'un dispositif immunochromatographique, dans lequel la partie d'échantillon du dispositif est connectée de manière fonctionnelle à une partie de capture espacée du dispositif et moyennant quoi les composants de l'échantillon s'écoulent de la partie d'échantillon du dispositif, et dans lequel une partie de capture comprend l'agent de liaison qui se lie spécifiquement à l'antigène dans l'échantillon de telle sorte que l'antigène est capturé par l'agent de liaison pour former un complexe agent de liaison-antigène dans la partie de capture ; éventuellement dans lequel la partie de capture est une ligne de test.

5. Dosage selon l'une quelconque des revendications 1 à 4, dans lequel le complexe antigénique est détecté à l'aide d'un agent de liaison tel qu'un anticorps ou un fragment de liaison à l'antigène, un ligand ou un affimère qui se lie spécifiquement à l'antigène et fournit directement ou indirectement un signal détectable qui peut être quantifié visuellement ou par un lecteur d'instrument.

6. Kit pour mesurer la concentration massique d'ALT1 dans un échantillon de sang d'un sujet humain comprenant (i) un dispositif chromatographique comprenant une membrane poreuse, ou un dispositif microfluidique connecté de manière fonctionnelle à une partie d'échantillon, une ou plusieurs parties de capture (test), et éventuellement l'un ou plusieurs des éléments suivants : une partie conjuguée (marqueur de détection), un composant de succion, une partie de commande adaptée et éventuellement une partie de lyse cellulaire ou de solubilisation cellulaire, et (ii) un anticorps lagomorphe ou un agent comprenant la partie de liaison à l'antigène de celui-ci qui reconnaît un épitope d'ALT1 humain qui n'est pas reconnu par des anticorps de rongeurs dirigés contre l'ALT1 humain, et forme un complexe ALT1-anticorps lagomorphe/partie de liaison, et (iii) éventuellement des instructions pour utiliser le dispositif pour déterminer la fonction hépatique ; éventuellement dans lequel la partie de capture est une ligne de test.

7. Procédé *in vitro* de criblage d'anticorps qui ne sont sensiblement pas inhibés par la liaison à ALT1 par le plasma humain, le procédé comprenant la mise en contact d'ALT1 humain et d'un anticorps potentiel en présence de différentes concentrations de plasma humain pour déterminer si l'anticorps peut se lier à l'antigène en présence de plasma, dans lequel l'anticorps est un anticorps lagomorphe ou comprend une partie de liaison à l'antigène de celui-ci.

8. Dosage selon l'une quelconque des revendications 1 à 5 comprenant également la détection des niveaux de plaquettes et la détermination de la fonction hépatique chez le sujet en fonction de la concentration massique d'enzymes hépatiques et des niveaux de plaquettes dans l'échantillon.

9. Kit selon la revendication 6 comprenant également des réactifs pour évaluer les niveaux de plaquettes.

10. Kit comprenant (i) un dispositif chromatographique comprenant une membrane poreuse ou un dispositif microfluidique connecté de manière fonctionnelle à une partie d'échantillon, deux ou plusieurs parties de capture (test), et éventuellement l'un ou plusieurs des éléments suivants : une partie conjuguée (marqueur de détection), une partie ventouse, une partie de commande adaptée et éventuellement une partie de lyse cellulaire ou de solubilisation cellulaire, et (ii) un agent de liaison spécifique qui reconnaît un épitope d'ALT1 ou d'une autre enzyme hépatique ou métabolite de celle-ci, qui n'est pas reconnu par ou en compétition avec les anticorps de rongeurs lorsque l'enzyme hépatique ou le métabolite sont en présence de plasma humain, et forme un complexe enzyme hépatique-anticorps/agent de liaison, et un second agent de liaison qui se lie spécifiquement aux plaquettes dans l'échantillon et forme un complexe marqueur plaquettaire-agent de liaison dans lequel les agents de liaison sont soit immobilisés pour séparer les parties de capture et/ou contenus dans les parties conjuguées et (iii) éventuellement des instructions pour utiliser le dispositif pour déterminer l'indice de rapport enzyme hépatique-plaquettes comme mesure de la fonction hépatique/fibrose et dans lequel l'agent de liaison spécifique est un anticorps lagomorphe ou comprend une partie de liaison à l'antigène de celui-ci, ou dans lequel l'agent de liaison spécifique reconnaît le même épitope que l'anticorps lagomorphe.
